# EUROPEAN PATENT APPLICATION

(11) **EP 1 577 322 A1**
(43) Date of publication of application: **21.09.2005**
(21) Application number: 03768194.7
(22) Date of filing: 25.12.2003
(51) Int. Cl.: C07K 14/47, C12N 15/12, C12P 21/02, C12Q 1/68, C07K 16/18, A01K 67/027, C12N 5/10, G01N 33/15, G01N 33/50, A61K 31/711, A61K 38/17, A61K 39/395, A61P 35/00

(54) **NOVEL PROTEINS AND USE THEREOF**

(30) Priority: 26.12.2002 JP 2002378052; 11.03.2003 JP 2003065497
(71) Applicant: Takeda Pharmaceutical Company Limited, Osaka 541-0045 (JP)
(72) Inventor: SUNAHARA, Eiji c/o Takeda Pharm.Co. LTD, Yodogawa-ku, Osaka-shi, Osaka 532-8686 (JP); ISHII, Takafumi, c/o Takeda Pharm. Co Ltd., Osaka-shi, Osaka 532-8686 (JP); YAMAMOTO, Koji c/o Takeda Pharm. Co. LTD., Ibaraki 300-4293 (JP); SATO, Shuji c/o Takeda Pharm. Co. Ltd., Osaka-shi, Osaka 532-8686 (JP)
(74) Representative: Rickard, Timothy Mark Adrian
(86) International application number: PCT/JP2003/016655
(87) International publication number: WO 2004/058817

(57) **Abstract**

Compounds that inhibit the expression of a protein comprising the same or substantially the same amino acid sequence as the amino acid sequence represented by SEQ ID NO: 1, SEQ ID NO: 4, SEQ ID NO: 7 or SEQ ID NO: 10 or the expression of a gene for the protein, the antisense polynucleotide comprising the entire or part of a base sequence complementary or substantially complementary to DNA encoding the protein or its partial peptide, the antibody to the protein or its partial peptide, etc. are useful as prophylactic/therapeutic agents for cancer, etc., apoptosis promoters, etc.

SEQUENCE LISTING

<110> Takeda Pharmaceutical Company Limited

<120> Novel protein and its use

<130> 3132WOOP

<150> JP2002-378052
<151> 2002-12-26
<150> JP2003-65497
<151> 2003-03-11

<160> 25

<210> 1
<211> 837
<212> PRT
<213> Homo sapiens

<400> 1

Met Leu Arg Thr Ala Met Gly Leu Arg Ser Trp Leu Ala Ala Pro Trp
                5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Met Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Lys Asp Val Gln Arg
        355                 360                 365

Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
                420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
                580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 2
<211> 2511
<212> DNA
<213> Homo sapiens

<400> 2

atgctgcgca ccgcgatggg cctgaggagc tggctcgccg ccccatgggg cgcgctgccg 60
cctcggccac cgctgctgct gctcctgctg ctgctgctcc tgctgcagcc gccgcctccg 120
acctgggcgc tcagcccccg gatcagcctg cctctgggct ctgaagagcg gccattcctc 180
agattcgaag ctgaacacat ctccaactac acagcccttc tgctgagcag ggatggcagg 240
accctgtacg tgggtgctcg agaggccctc tttgcactca gtagcaacct cagcttcctg 300
ccaggcgggg agtaccagga gctgctttgg ggtgcagacg cagagaagaa acagcagtgc 360
agcttcaagg gcaaggaccc acagcgcgac tgtcaaaact acatcaagat cctcctgccg 420
ctcagcggca gtcacctgtt cacctgtggc acagcagcct tcagccccat gtgtacctac 480
atcaacatgg agaacttcac cctggcaagg gacgagaagg ggaatgtcct cctggaagat 540
ggcaagggcc gttgtccctt cgacccgaat ttcaagtcca ctgccctggt ggttgatggc 600
gagctctaca ctggaacagt cagcagcttc caagggaatg acccggccat ctcgcggagc 660
caaagccttc gccccaccaa gaccgagagc tccctcaact ggctgcaaga cccagctttt 720
gtggcctcag cctacattcc tgagagcctg ggcagcttgc aaggcgatga tgacaagatc 780
tactttttct tcagcgagac tggccaggaa tttgagttct ttgagaacac cattgtgtcc 840
cgcattgccc gcatctgcaa gggcgatgag ggtggagagc gggtgctaca gcagcgctgg 900
acctccttcc tcaaggccca gctgctgtgc tcacggcccg acgatggctt ccccttcaac 960
gtgctgcagg atgtcttcac gctgagcccc agcccccagg actggcgtga cacccttttc 1020
tatggggtct tcacttccca gtggcacagg ggaactacag aaggctctgc cgtctgtgtc 1080
ttcacaatga aggatgtgca gagagtcttc agcggcctct acaaggaggt gaaccgtgag 1140
acacagcaga tggtacaccg tgacccaccc gtgcccacac cccggcctgg agcgtgcatc 1200
accaacagtg cccgggaaag gaagatcaac tcatccctgc agctcccaga ccgcgtgctg 1260
aactttctca aggaccactt cctgatggac gggcaggtcc gaagccgcat gctgctgctg 1320
cagccccagg ctcgctacca gcgcgtggct gtacaccgcg tccctggcct gcaccacacc 1380
tacgatgtcc tcttcctggg cactggtgac ggccggctcc acaaggcagt gagcgtgggc 1440
ccccgggtgc acatcattga ggagctgcag atcttctcat cgggacagcc cgtgcagaat 1500
ctgctcctgg acacccacag gggctgctg tatgcggcct cacactcggg cgtagtccag 1560
gtgcccatgg ccaactgcag cctgtaccgg agctgtgggg actgcctcct cgcccgggac 1620
ccctactgtg cttggagcgg ctccagctgc aagcacgtca gcctctacca gcctcagctg 1680
gccaccaggc cgtggatcca ggacatcgag ggagccagcg ccaaggacct ttgcagcgcg 1740
tcttcggttg tgtccccgtc ttttgtacca acaggggaga agccatgtga gcaagtccag 1800
ttccagccca acacagtgaa cactttggcc tgcccgctcc tctccaacct ggcgacccga 1860
ctctggctac gcaacggggc ccccgtcaat gcctcggcct cctgccacgt gctacccact 1920
ggggacctgc tgctggtggg cacccaacag ctgggggagt tccagtgctg gtcactagag 1980
gagggcttcc agcagctggt agccagctac tgcccagagg tggtggagga cggggtggca 2040
gaccaaacag atgagggtgg cagtgtaccc gtcattatca gcacatcgcg tgtgagtgca 2100
ccagctggtg gcaaggccag ctggggtgca gacaggtcct actggaagga gttcctggtg 2160
atgtgcacgc tctttgtgct ggccgtgctg ctcccagttt tattcttgct ctaccggcac 2220
cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccactcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 3
<211> 3766
<212> DNA
<213> Homo sapiens

<400> 3

gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctgctg ctgctcctgc tgcagccgcc gcctccgacc 360
tgggcgctca gcccccggat cagcctgcct ctgggctctg aagagcggcc attcctcaga 420
ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatggaga acttcaccct ggcaagggac gagaagggga atgtcctcct ggaagatggc 780
aagggccgtt gtcccttcga cccgaatttc aagtccactg ccctggtggt tgatggcgag 840
ctctacactg gaacagtcag cagcttccaa gggaatgacc cggccatctc gcggagccaa 900
agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080

attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc cccccaggact ggcgtgacac cctttttctat 1260
ggggtcttca cttcccagtg gcacagggga actacagaag gctctgccgt ctgtgtcttc 1320
acaatgaagg atgtgcagag agtcttcagc ggcctctaca aggaggtgaa ccgtgagaca 1380
cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
tttctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtaccggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg ggggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcacccca 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300
ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540
ccaccatatc caccctcgct ccatctttga actcaaacac gaggaactaa ctgcaccctg 3600
gtcctctccc cagtccccag ttcaccctcc atccctcacc ttcctccact ctaagggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 4
<211> 837
<212> PRT
<213> Homo sapiens

<400> 4

Met Leu Arg Thr Ala Met Gly Leu Arg Ser Trp Leu Ala Ala Pro Trp
1               5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln

        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Met Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ile
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Lys Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro

                660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 5
<211> 2511
<212> DNA
<213> Homo sapiens

<400> 5

atgctgcgca ccgcgatggg cctgaggagc tggctcgccg ccccatgggg cgcgctgccg      60
cctcggccac cgctgctgct gctcctgcta ctgctgctcc tgctgcagcc accgcctccg     120
acctgggcgc tcagcccccg gatcagcctg cctctgggct ctgaagagcg gccattcctc     180
agattcgaag ctgaacacat ctccaactac acagcccttc tgctgagcag ggatggcagg     240
accctgtacg tgggtgctcg agaggccctc tttgcactca gtagcaacct cagcttcctg     300
ccaggcgggg agtaccagga gctgctttgg ggtgcagacg cagagaagaa acagcagtgc     360
agcttcaagg gcaaggaccc acagcgcgac tgtcaaaact acatcaagat cctcctgccg     420
ctcagcggca gtcacctgtt cacctgtggc acagcagcct tcagccccat gtgtacctac     480
atcaacatgg agaacttcac cctggcaagg gacgagaagg ggaatgtcct cctggaagat     540
ggcaagggcc gttgtccctt cgacccgaat ttcaagtcca ctgccctggt ggttgatggc     600
gagctctaca ctggaacagt catcagcttc caagggaatg acccggccat ctcgcggagc     660
caaagccttc gccccaccaa gaccgagagc tccctcaact ggctgcaaga cccagctttt     720
gtggcctcag cctacattcc tgagagcctg ggcagcttgc aaggcgatga tgacaagatc     780
tactttttct tcagcgagac tggccaggaa tttgagttct ttgagaacac cattgtgtcc     840
cgcattgccc gcatctgcaa gggcgatgag ggtggagagc gggtgctaca gcagcgctgg     900
acctccttcc tcaaggccca gctgctgtgc tcacggcccg acgatggctt ccccttcaac     960
gtgctgcagg atgtcttcac gctgagcccc agcccccagg actggcgtga cacccttttc     1020
tatggggtct tcacttccca gtggcacagg ggaactacag aaggctctgc cgtctgtgtc    1080
ttcacaatga aggatgtgca gagagtcttc agcggcctct acaaggaggt gaaccgtgag    1140
acacagcaga tggtacaccg tgacccaccc gtgcccacac cccggcctgg agcgtgcatc    1200
accaacagtg cccgggaaag gaagatcaac tcatccctgc agctcccaga ccgcgtgctg    1260
aactttctca aggaccactt cctgatggac gggcaggtcc gaagccgcat gctgctgctg    1320
cagccccagg ctcgctacca gcgcgtggct gtacaccgcg tccctggcct gcaccacacc    1380
tacgatgtcc tcttcctggg cactggtgac ggccggctcc acaaggcagt gagcgtgggc    1440
ccccgggtgc acatcattga ggagctgcag atcttctcat cgggacagcc cgtgcagaat    1500
ctgctcctgg acacccacag gggctgctg tatgcggcct cacactcggg cgtagtccag     1560
gtgcccatgg ccaactgcag cctgtaccgg agctgtgggg actgcctcct cgcccgggac    1620
ccctactgtg cttggagcgg ctccagctgc aagcacgtca gcctctacca gcctcagctg    1680
gccaccaggc cgtggatcca ggacatcgag ggagccagcg ccaaggacct ttgcagcgcg    1740
tcttcggttg tgtccccgtc ttttgtacca acaggggaga agccatgtga gcaagtccag    1800
ttccagccca acacagtgaa cactttggcc tgcccgctcc tctccaacct ggcgacccga    1860
ctctggctac gcaacggggc ccccgtcaat gcctcggcct cctgccacgt gctacccact    1920
ggggacctgc tgctggtggg cacccaacag ctgggggagt tccagtgctg gtcactagag    1980
gagggcttcc agcagctggt agccagctac tgcccagagg tggtggagga cggggtggca    2040
gaccaaacag atgagggtgg cagtgtaccc gtcattatca gcacatcgcg tgtgagtgca    2100
ccagctggtg gcaaggccag ctggggtgca gacaggtcct actggaagga gttcctggtg    2160
atgtgcacgc tctttgtgct ggccgtgctg ctcccagttt tattcttgct ctaccggcac    2220

cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccactcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 6
<211> 3766
<212> DNA
<213> Homo sapiens

<400> 6

gctctgccca agccgaggct gcggggccgg cgccggcggg aggactgcgg tgccccgcgg 60
aggggctgag tttgccaggg cccacttgac cctgtttccc acctcccgcc ccccaggtcc 120
ggaggcgggg gcccccgggg cgactcgggg gcggaccgcg gggcggagct gccgcccgtg 180
agtccggccg agccacctga gcccgagccg cgggacaccg tcgctcctgc tctccgaatg 240
ctgcgcaccg cgatgggcct gaggagctgg ctcgccgccc catggggcgc gctgccgcct 300
cggccaccgc tgctgctgct cctgctactg ctgctcctgc tgcagccacc gcctccgacc 360
tgggcgctca gcccccggat cagcctgcct ctgggctctg aagagcggcc attcctcaga 420
ttcgaagctg aacacatctc caactacaca gcccttctgc tgagcaggga tggcaggacc 480
ctgtacgtgg gtgctcgaga ggccctcttt gcactcagta gcaacctcag cttcctgcca 540
ggcggggagt accaggagct gctttggggt gcagacgcag agaagaaaca gcagtgcagc 600
ttcaagggca aggacccaca gcgcgactgt caaaactaca tcaagatcct cctgccgctc 660
agcggcagtc acctgttcac ctgtggcaca gcagccttca gccccatgtg tacctacatc 720
aacatggaga acttcaccct ggcaagggac gagaagggga atgtcctcct ggaagatggc 780
aagggccgtt gtcccttcga cccgaatttc aagtccactg ccctggtggt tgatggcgag 840
ctctacactg gaacagtcat cagcttccaa gggaatgacc cggccatctc gcggagccaa 900
agccttcgcc ccaccaagac cgagagctcc ctcaactggc tgcaagaccc agcttttgtg 960
gcctcagcct acattcctga gagcctgggc agcttgcaag gcgatgatga caagatctac 1020
tttttcttca gcgagactgg ccaggaattt gagttctttg agaacaccat tgtgtcccgc 1080
attgcccgca tctgcaaggg cgatgagggt ggagagcggg tgctacagca gcgctggacc 1140
tccttcctca aggcccagct gctgtgctca cggcccgacg atggcttccc cttcaacgtg 1200
ctgcaggatg tcttcacgct gagccccagc ccccaggact ggcgtgacac ccttttctat 1260
ggggtcttca cttcccagtg gcacagggga actacagaag gctctgccgt ctgtgtcttc 1320
acaatgaagg atgtgcagag agtcttcagc ggcctctaca aggaggtgaa ccgtgagaca 1380
cagcagatgg tacaccgtga cccacccgtg cccacacccc ggcctggagc gtgcatcacc 1440
aacagtgccc gggaaaggaa gatcaactca tccctgcagc tcccagaccg cgtgctgaac 1500
tttctcaagg accacttcct gatggacggg caggtccgaa gccgcatgct gctgctgcag 1560
ccccaggctc gctaccagcg cgtggctgta caccgcgtcc ctggcctgca ccacacctac 1620
gatgtcctct tcctgggcac tggtgacggc cggctccaca aggcagtgag cgtgggcccc 1680
cgggtgcaca tcattgagga gctgcagatc ttctcatcgg gacagcccgt gcagaatctg 1740
ctcctggaca cccacagggg gctgctgtat gcggcctcac actcgggcgt agtccaggtg 1800
cccatggcca actgcagcct gtaccggagc tgtggggact gcctcctcgc ccgggacccc 1860
tactgtgctt ggagcggctc cagctgcaag cacgtcagcc tctaccagcc tcagctggcc 1920
accaggccgt ggatccagga catcgaggga gccagcgcca aggacctttg cagcgcgtct 1980
tcggttgtgt ccccgtcttt tgtaccaaca ggggagaagc catgtgagca agtccagttc 2040
cagcccaaca cagtgaacac tttggcctgc ccgctcctct ccaacctggc gacccgactc 2100
tggctacgca acggggcccc cgtcaatgcc tcggcctcct gccacgtgct acccactggg 2160
gacctgctgc tggtgggcac ccaacagctg ggggagttcc agtgctggtc actagaggag 2220
ggcttccagc agctggtagc cagctactgc ccagaggtgg tggaggacgg ggtggcagac 2280
caaacagatg agggtggcag tgtacccgtc attatcagca catcgcgtgt gagtgcacca 2340
gctggtggca aggccagctg gggtgcagac aggtcctact ggaaggagtt cctggtgatg 2400
tgcacgctct ttgtgctggc cgtgctgctc ccagttttat tcttgctcta ccggcaccgg 2460
aacagcatga aagtcttcct gaagcagggg gaatgtgcca gcgtgcaccc caagacctgc 2520
cctgtggtgc tgccccctga gacccgccca ctcaacggcc tagggccccc tagcacccca 2580
ctcgatcacc gagggtacca gtccctgtca gacagccccc cggggtcccg agtcttcact 2640
gagtcagaga agaggccact cagcatccaa gacagcttcg tggaggtatc cccagtgtgc 2700
ccccggcccc gggtccgcct tggctcggag atccgtgact ctgtggtgtg agagctgact 2760
tccagaggac gctgccctgg cttcaggggc tgtgaatgct cggagagggt caactggacc 2820
tcccctccgc tctgctcttc gtggaacacg accgtggtgc ccggcccttg ggagccttgg 2880
ggccagctgg cctgctgctc tccagtcaag tagcgaagct cctaccaccc agacacccaa 2940
acagccgtgg ccccagaggt cctggccaaa tatgggggcc tgcctaggtt ggtggaacag 3000
tgctccttat gtaaactgag ccctttgttt aaaaaacaat tccaaatgtg aaactagaat 3060
gagagggaag agatagcatg gcatgcagca cacacggctg ctccagttca tggcctccca 3120
ggggtgctgg ggatgcatcc aaagtggttg tctgagacag agttggaaac cctcaccaac 3180
tggcctcttc accttccaca ttatcccgct gccaccggct gccctgtctc actgcagatt 3240
caggaccagc ttgggctgcg tgcgttctgc cttgccagtc agccgaggat gtagttgttg 3300

ctgccgtcgt cccaccacct cagggaccag agggctaggt tggcactgcg gccctcacca 3360
ggtcctgggc tcggacccaa ctcctggacc tttccagcct gtatcaggct gtggccacac 3420
gagaggacag cgcgagctca ggagagattt cgtgacaatg tacgcctttc cctcagaatt 3480
cagggaagag actgtcgcct gccttcctcc gttgttgcgt gagaacccgt gtgccccttc 3540
ccaccatatc caccctcgct ccatctttga actcaaacac gaggaactaa ctgcaccctg 3600
gtcctctccc cagtccccag ttcaccctcc atccctcacc ttcctccact ctaagggata 3660
tcaacactgc ccagcacagg ggccctgaat ttatgtggtt tttatacatt ttttaataag 3720
atgcacttta tgtcattttt taataaagtc tgaagaatta ctgttt 3766

<210> 7
<211> 837
<212> PRT
<213> Homo sapiens

<400> 7

Met Leu Arg Thr Ala Met Gly Leu Arg Ser Trp Leu Ala Ala Pro Trp
1               5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Ile Glu Asn Phe Thr Leu Ala Arg Asp Glu Lys Gly Asn Val
                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Lys Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415

Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val
705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 8
<211> 2511
<212> DNA
<213> Homo sapiens

<400> 8

atgctgcgca ccgcgatggg cctgaggagc tggctcgccg ccccatgggg cgcgctgccg      60
cctcggccac cgctgctgct gctcctgctg ctgctgctcc tgctgcagcc gccgcctccg     120
acctgggcgc tcagcccccg gatcagccta cctctgggct ctgaagagcg gccattcctc     180
agattcgaag ctgaacacat ctccaactac acagcccttc tgctgagcag ggatggcagg     240
accctgtacg tgggtgctcg agaggccctc tttgcactca gtagcaacct cagcttcctg     300
ccaggcgggg agtaccagga gctgctttgg ggtgcagacg cagagaagaa acagcagtgc     360

agcttcaagg gcaaggaccc acagcgcgac tgtcaaaact acatcaagat cctcctgccg 420
ctcagcggca gtcacctgtt cacctgtggc acagcagcct tcagccccat gtgtacctac 480
atcaacatag agaacttcac cctggcaagg gacgagaagg ggaatgttct cctggaagat 540
ggcaagggcc gttgtccctt cgacccgaat ttcaagtcca ctgccctggt ggttgatggc 600
gagctctaca ctggaacagt cagcagcttc caagggaatg acccggccat ctcgcggagc 660
caaagccttc gccccaccaa gaccgagagc tccctcaact ggctgcaaga cccagctttt 720
gtggcctcag cctacattcc tgagagcctg ggcagcttgc aaggcgatga tgacaagatc 780
tactttttct tcagcgagac tggccaggaa tttgagttct ttgagaacac cattgtgtcc 840
cgcattgccc gcatctgcaa gggcgatgag ggtggagagc gggtgctaca gcagcgctgg 900
acctccttcc tcaaggccca gctgctgtgc tcacggcccg acgatggctt ccccttcaac 960
gtgctgcagg atgtcttcac gctgagcccc agcccccagg actggcgtga cacccttttc 1020
tatggggtct tcacttccca gtggcacagg ggaactacag aaggctctgc cgtctgtgtc 1080
ttcacaatga aggatgtgca gagagtcttc agcggcctct acaaggaggt gaaccgtgag 1140
acacagcaga tggtacaccg tgacccaccc gtgcccacac cccggcctgg agcgtgcatc 1200
accaacagtg cccgggaaag gaagatcaac tcatccctgc agctcccaga ccgcgtgctg 1260
aacttcctca aggaccactt cctgatggac gggcaggtcc gaagccgcat gctgctgctg 1320
cagccccagg ctcgctacca gcgcgtggct gtacaccgcg tccctggcct gcaccacacc 1380
tacgatgtcc tcttcctggg cactggtgac ggccggctcc acaaggcagt gagcgtgggc 1440
ccccgggtgc acatcattga ggagctgcag atcttctcat cgggacagcc cgtgcagaat 1500
ctgctcctgg acacccacag ggggctgctg tatgcggcct cacactcggg cgtagtccag 1560
gtgcccatgg ccaactgcag cctgtacagg agctgtgggg actgcctcct cgcccgggac 1620
ccctactgtg cttggagcgg ctccagctgc aagcacgtca gcctctacca gcctcagctg 1680
gccaccaggc cgtggatcca ggacatcgag ggagccagcg ccaaggacct ttgcagcgcg 1740
tcttcggttg tgtccccgtc ttttgtacca acaggggaga agccatgtga gcaagtccag 1800
ttccagccca acacagtgaa cactttggcc tgcccgctcc tctccaacct ggcgacccga 1860
ctctggctac gcaacggggc ccccgtcaat gcctcggcct cctgccacgt gctacccact 1920
ggggacctgc tgctggtggg cacccaacag ctgggggagt tccagtgctg gtcactagag 1980
gagggcttcc agcagctggt agccagctac tgcccagagg tggtggagga cggggtggca 2040
gaccaaacag atgagggtgg cagtgtaccc gtcattatca gcacatcgcg tgtgagtgca 2100
ccagctggtg gcaaggccag ctggggtgca gacaggtcct actggaagga gttcctggtg 2160
atgtgcacgc tctttgtgct ggccgtgctg ctcccagttt tattcttgct ctaccggcac 2220
cggaacagca tgaaagtctt cctgaagcag ggggaatgtg ccagcgtgca ccccaagacc 2280
tgccctgtgg tgctgccccc tgagacccgc ccactcaacg gcctagggcc ccctagcacc 2340
ccgctcgatc accgagggta ccagtccctg tcagacagcc ccccggggtc ccgagtcttc 2400
actgagtcag agaagaggcc actcagcatc caagacagct tcgtggaggt atccccagtg 2460
tgcccccggc cccgggtccg ccttggctcg gagatccgtg actctgtggt g 2511

<210> 9
<211> 3766
<212> DNA
<213> Homo sapiens

<400> 9
   gctctgccca agccgaggct gcggggccgg cgccggcggg aggactgcgg tgccccgcgg 60
   aggggctgag tttgccaggg cccacttgac cctgtttccc acctcccgcc ccccaggtcc 120
   ggaggcgggg gcccccgggg cgactcgggg gcggaccgcg gggcggagct gccgcccgtg 180
   agtccggccg agccacctga gcccgagccg cgggacaccg tcgctcctgc tctccgaatg 240
   ctgcgcaccg cgatgggcct gaggagctgg ctcgccgccc catggggcgc gctgccgcct 300
   cggccaccgc tgctgctgct cctgctgctg ctgctcctgc tgcagccgcc gcctccgacc 360
   tgggcgctca gcccccggat cagcctacct ctgggctctg aagagcggcc attcctcaga 420
   ttcgaagctg aacacatctc caactacaca gcccttctgc tgagcaggga tggcaggacc 480
   ctgtacgtgg gtgctcgaga ggccctcttt gcactcagta gcaacctcag cttcctgcca 540
   ggcggggagt accaggagct gctttggggt gcagacgcag agaagaaaca gcagtgcagc 600
   ttcaagggca aggacccaca gcgcgactgt caaaactaca tcaagatcct cctgccgctc 660
   agcggcagtc acctgttcac ctgtggcaca gcagccttca gccccatgtg tacctacatc 720
   aacatagaga acttcaccct ggcaagggac gagaagggga atgttctcct ggaagatggc 780
   aagggccgtt gtcccttcga cccgaatttc aagtccactg ccctggtggt tgatggcgag 840
   ctctacactg gaacagtcag cagcttccaa gggaatgacc cggccatctc gcggagccaa 900
   agccttcgcc ccaccaagac cgagagctcc ctcaactggc tgcaagaccc agcttttgtg 960
   gcctcagcct acattcctga gagcctgggc agcttgcaag gcgatgatga caagatctac 1020
   tttttcttca gcgagactgg ccaggaattt gagttctttg agaacaccat tgtgtcccgc 1080
   attgcccgca tctgcaaggg cgatgagggt ggagagcggg tgctacagca gcgctggacc 1140
   tccttcctca aggcccagct gctgtgctca cggcccgacg atggcttccc cttcaacgtg 1200
   ctgcaggatg tcttcacgct gagccccagc ccccaggact ggcgtgacac ccttttctat 1260
   ggggtcttca cttcccagtg gcacagggga actacagaag gctctgccgt ctgtgtcttc 1320
   acaatgaagg atgtgcagag agtcttcagc ggcctctaca aggaggtgaa ccgtgagaca 1380
   cagcagatgg tacaccgtga cccacccgtg cccacacccc ggcctggagc gtgcatcacc 1440
   aacagtgccc gggaaaggaa gatcaactca tccctgcagc tcccagaccg cgtgctgaac 1500
   ttcctcaagg accacttcct gatggacggg caggtccgaa gccgcatgct gctgctgcag 1560
   ccccaggctc gctaccagcg cgtggctgta caccgcgtcc ctggcctgca ccacacctac 1620
   gatgtcctct tcctgggcac tggtgacggc cggctccaca aggcagtgag cgtgggcccc 1680
   cgggtgcaca tcattgagga gctgcagatc ttctcatcgg gacagcccgt gcagaatctg 1740
   ctcctggaca cccacagggg gctgctgtat gcggcctcac actcgggcgt agtccaggtg 1800
   cccatggcca actgcagcct gtacaggagc tgtggggact gcctcctcgc ccgggacccc 1860
   tactgtgctt ggagcggctc cagctgcaag cacgtcagcc tctaccagcc tcagctggcc 1920
   accaggccgt ggatccagga catcgaggga gccagcgcca aggacctttg cagcgcgtct 1980
   tcggttgtgt ccccgtcttt tgtaccaaca ggggagaagc catgtgagca agtccagttc 2040
   cagcccaaca cagtgaacac tttggcctgc ccgctcctct ccaacctggc gacccgactc 2100
   tggctacgca acggggcccc cgtcaatgcc tcggcctcct gccacgtgct acccactggg 2160
   gacctgctgc tggtgggcac ccaacagctg ggggagttcc agtgctggtc actagaggag 2220
   ggcttccagc agctggtagc cagctactgc ccagaggtgg tggaggacgg ggtggcagac 2280
   caaacagatg agggtggcag tgtacccgtc attatcagca catcgcgtgt gagtgcacca 2340
   gctggtggca aggccagctg gggtgcagac aggtcctact ggaaggagtt cctggtgatg 2400
   tgcacgctct ttgtgctggc cgtgctgctc ccagttttat tcttgctcta ccggcaccgg 2460
   aacagcatga aagtcttcct gaagcagggg gaatgtgcca gcgtgcaccc caagacctgc 2520
   cctgtggtgc tgccccctga gacccgccca ctcaacggcc tagggccccc tagcaccccg 2580
   ctcgatcacc gagggtacca gtccctgtca gacagccccc cggggtcccg agtcttcact 2640
   gagtcagaga agaggccact cagcatccaa gacagcttcg tggaggtatc cccagtgtgc 2700
   ccccggcccc gggtccgcct tggctcggag atccgtgact ctgtggtgtg agagctgact 2760
   tccagaggac gctgccctgg cttcaggggc tgtgaatgct cggagagggt caactggacc 2820
   tcccctccgc tctgctcttc gtggaacacg accgtggtgc ccggcccttg ggagccttgg 2880
   ggccagctgg cctgctgctc tccagtcaag tagcgaagct cctaccaccc agacacccaa 2940
   acagccgtgg ccccagaggt cctggccaaa tatgggggcc tgcctaggtt ggtggaacag 3000
   tgctccttat gtaaactgag ccctttgttt aaaaaacaat tccaaatgtg aaactagaat 3060
   gagagggaag agatagcatg gcatgcagca cacacggctg ctccagttca tggcctccca 3120
   ggggtgctgg ggatgcatcc aaagtggttg tctgagacag agttggaaac cctcaccaac 3180
   tggcctcttc accttccaca ttatcccgct gccaccggct gccctgtctc actgcagatt 3240
   caggaccagc ttgggctgcg tgcgttctgc cttgccagtc agccgaggat gtagttgttg 3300
   ctgccgtcgt cccaccacct cagggaccag agggctaggt tggcactgcg gccctcacca 3360
   ggtcctgggc tcggacccaa ctcctggacc tttccagcct gtatcaggct gtggccacac 3420
   gagaggacag cgcgagctca ggagagattt cgtgacaatg tacgcctttc cctcagaatt 3480
   cagggaagag actgtcgcct gccttcctcc gttgttgcgt gagaacccgt gtgccccttc 3540
   ccaccatatc caccctcgct ccatctttga actcaaacac gaggaactaa ctgcaccctg 3600
   gtcctctccc cagtccccag ttcaccctcc atccctcacc ttcctccact ctaagggata 3660
   tcaacactgc ccagcacagg ggccctgaat ttatgtggtt tttatacatt ttttaataag 3720
   atgcacttta tgtcattttt taataaagtc tgaagaatta ctgttt 3766

<210> 10
<211> 837
<212> PRT
<213> Homo sapiens

<400> 10

Met Leu Arg Thr Ala Met Gly Leu Arg Ser Trp Leu Ala Ala Pro Trp
1               5                   10                  15
Gly Ala Leu Pro Pro Arg Pro Pro Leu Leu Leu Leu Leu Leu Leu Leu
            20                  25                  30
Leu Leu Leu Gln Pro Pro Pro Pro Thr Trp Ala Leu Ser Pro Arg Ile
        35                  40                  45
Ser Leu Pro Leu Gly Ser Glu Glu Arg Pro Phe Leu Arg Phe Glu Ala
    50                  55                  60
Glu His Ile Ser Asn Tyr Thr Ala Leu Leu Leu Ser Arg Asp Gly Arg
65                  70                  75                  80
Thr Leu Tyr Val Gly Ala Arg Glu Ala Leu Phe Ala Leu Ser Ser Asn
                85                  90                  95
Leu Ser Phe Leu Pro Gly Gly Glu Tyr Gln Glu Leu Leu Trp Gly Ala
            100                 105                 110
Asp Ala Glu Lys Lys Gln Gln Cys Ser Phe Lys Gly Lys Asp Pro Gln
        115                 120                 125
Arg Asp Cys Gln Asn Tyr Ile Lys Ile Leu Leu Pro Leu Ser Gly Ser
    130                 135                 140
His Leu Phe Thr Cys Gly Thr Ala Ala Phe Ser Pro Met Cys Thr Tyr
145                 150                 155                 160
Ile Asn Met Glu Asn Phe Thr Leu Ala Arg Asp Glu Lys Gly Asn Val

                165                 170                 175
Leu Leu Glu Asp Gly Lys Gly Arg Cys Pro Phe Asp Pro Asn Phe Lys
            180                 185                 190
Ser Thr Ala Leu Val Val Asp Gly Glu Leu Tyr Thr Gly Thr Val Ser
        195                 200                 205
Ser Phe Gln Gly Asn Asp Pro Ala Ile Ser Arg Ser Gln Ser Leu Arg
    210                 215                 220
Pro Thr Lys Thr Glu Ser Ser Leu Asn Trp Leu Gln Asp Pro Ala Phe
225                 230                 235                 240
Val Ala Ser Ala Tyr Ile Pro Glu Ser Leu Gly Ser Leu Gln Gly Asp
                245                 250                 255
Asp Asp Lys Ile Tyr Phe Phe Phe Ser Glu Thr Gly Gln Glu Phe Glu
            260                 265                 270
Phe Phe Glu Asn Thr Ile Val Ser Arg Ile Ala Arg Ile Cys Lys Gly
        275                 280                 285
Asp Glu Gly Gly Glu Arg Val Leu Gln Gln Arg Trp Thr Ser Phe Leu
    290                 295                 300
Lys Ala Gln Leu Leu Cys Ser Arg Pro Asp Asp Gly Phe Pro Phe Asn
305                 310                 315                 320
Val Leu Gln Asp Val Phe Thr Leu Ser Pro Ser Pro Gln Asp Trp Arg
                325                 330                 335
Asp Thr Leu Phe Tyr Gly Val Phe Thr Ser Gln Trp His Arg Gly Thr
            340                 345                 350
Thr Glu Gly Ser Ala Val Cys Val Phe Thr Met Asn Asp Val Gln Arg
        355                 360                 365
Val Phe Ser Gly Leu Tyr Lys Glu Val Asn Arg Glu Thr Gln Gln Met
    370                 375                 380
Val His Arg Asp Pro Pro Val Pro Thr Pro Arg Pro Gly Ala Cys Ile
385                 390                 395                 400
Thr Asn Ser Ala Arg Glu Arg Lys Ile Asn Ser Ser Leu Gln Leu Pro
                405                 410                 415
Asp Arg Val Leu Asn Phe Leu Lys Asp His Phe Leu Met Asp Gly Gln
            420                 425                 430
Val Arg Ser Arg Met Leu Leu Leu Gln Pro Gln Ala Arg Tyr Gln Arg
        435                 440                 445
Val Ala Val His Arg Val Pro Gly Leu His His Thr Tyr Asp Val Leu
    450                 455                 460
Phe Leu Gly Thr Gly Asp Gly Arg Leu His Lys Ala Val Ser Val Gly
465                 470                 475                 480
Pro Arg Val His Ile Ile Glu Glu Leu Gln Ile Phe Ser Ser Gly Gln
                485                 490                 495
Pro Val Gln Asn Leu Leu Leu Asp Thr His Arg Gly Leu Leu Tyr Ala
            500                 505                 510
Ala Ser His Ser Gly Val Val Gln Val Pro Met Ala Asn Cys Ser Leu
        515                 520                 525
Tyr Arg Ser Cys Gly Asp Cys Leu Leu Ala Arg Asp Pro Tyr Cys Ala
    530                 535                 540
Trp Ser Gly Ser Ser Cys Lys His Val Ser Leu Tyr Gln Pro Gln Leu
545                 550                 555                 560
Ala Thr Arg Pro Trp Ile Gln Asp Ile Glu Gly Ala Ser Ala Lys Asp
                565                 570                 575
Leu Cys Ser Ala Ser Ser Val Val Ser Pro Ser Phe Val Pro Thr Gly
            580                 585                 590
Glu Lys Pro Cys Glu Gln Val Gln Phe Gln Pro Asn Thr Val Asn Thr
        595                 600                 605
Leu Ala Cys Pro Leu Leu Ser Asn Leu Ala Thr Arg Leu Trp Leu Arg
    610                 615                 620
Asn Gly Ala Pro Val Asn Ala Ser Ala Ser Cys His Val Leu Pro Thr
625                 630                 635                 640
Gly Asp Leu Leu Leu Val Gly Thr Gln Gln Leu Gly Glu Phe Gln Cys
                645                 650                 655
Trp Ser Leu Glu Glu Gly Phe Gln Gln Leu Val Ala Ser Tyr Cys Pro
            660                 665                 670
Glu Val Val Glu Asp Gly Val Ala Asp Gln Thr Asp Glu Gly Gly Ser
        675                 680                 685
Val Pro Val Ile Ile Ser Thr Ser Arg Val Ser Ala Pro Ala Gly Gly
    690                 695                 700
Lys Ala Ser Trp Gly Ala Asp Arg Ser Tyr Trp Lys Glu Phe Leu Val

705                 710                 715                 720
Met Cys Thr Leu Phe Val Leu Ala Val Leu Leu Pro Val Leu Phe Leu
                725                 730                 735
Leu Tyr Arg His Arg Asn Ser Met Lys Val Phe Leu Lys Gln Gly Glu
            740                 745                 750
Cys Ala Ser Val His Pro Lys Thr Cys Pro Val Val Leu Pro Pro Glu
        755                 760                 765
Thr Arg Pro Leu Asn Gly Leu Gly Pro Pro Ser Thr Pro Leu Asp His
    770                 775                 780
Arg Gly Tyr Gln Ser Leu Ser Asp Ser Pro Pro Gly Ser Arg Val Phe
785                 790                 795                 800
Thr Glu Ser Glu Lys Arg Pro Leu Ser Ile Gln Asp Ser Phe Val Glu
                805                 810                 815
Val Ser Pro Val Cys Pro Arg Pro Arg Val Arg Leu Gly Ser Glu Ile
            820                 825                 830
Arg Asp Ser Val Val
        835

<210> 11
<211> 2511
<212> DNA
<213> Homo sapiens

<400> 11
   atgctgcgca ccgcgatggg cctgaggagc tggctcgccg ccccatgggg cgcgctgccg      60
   cctcggccac cgctgctgct gctcctgctg ctgctgctcc tgctgcagcc gccgcctccg     120
   acctgggcgc tcagcccccg gatcagcctg cctctgggct ctgaagagcg gccattcctc     180
   agattcgaag ctgaacacat ctccaactac acagcccttc tgctgagcag ggatggcagg     240
   accctgtacg tgggtgctcg agaggccctc tttgcactca gtagcaacct cagcttcctg     300
   ccaggcgggg agtaccagga gctgctttgg ggtgcagacg cagagaagaa acagcagtgc     360
   agcttcaagg gcaaggaccc acagcgcgac tgtcaaaact acatcaagat cctcctgccg     420
   ctcagcggca gtcacctgtt cacctgtggc acagcagcct tcagccccat gtgtacctac     480
   atcaacatgg agaacttcac cctggcaagg gacgagaagg ggaatgtcct cctggaagat     540
   ggcaagggcc gttgtccctt cgacccgaat ttcaagtcca ctgccctggt ggttgatggc     600
   gagctctaca ctggaacagt cagcagcttc caagggaatg acccggccat ctcgcggagc     660
   caaagccttc gccccaccaa gaccgagagc tccctcaact ggctgcaaga cccagctttt     720
   gtggcctcag cctacattcc tgagagcctg ggcagcttgc aaggcgatga tgacaagatc     780
   tactttttct tcagcgagac tggccaggaa tttgagttct ttgagaacac cattgtgtcc     840
   cgcattgccc gcatctgcaa gggcgatgag ggtggagagc gggtgctaca gcagcgctgg     900
   acctccttcc tcaaggccca gctgctgtgc tcacggcccg acgatggctt ccccttcaac     960
   gtgctgcagg atgtcttcac gctgagcccc agcccccagg actggcgtga cacccttttc     1020
   tatggggtct tcacttccca gtggcacagg ggaactacag aaggctctgc cgtctgtgtc    1080
   ttcacaatga atgatgtgca gagagtcttc agcggcctct acaaggaggt gaaccgtgag    1140
   acacagcaga tggtacaccg tgacccaccc gtgcccacac cccggcctgg agcgtgcatc    1200
   accaacagtg cccgggaaag gaagatcaac tcatccctgc agctcccaga ccgcgtgctg    1260
   aactttctca aggaccactt cctgatggac gggcaggtcc gaagccgcat gctgctgctg    1320
   cagccccagg ctcgctacca gcgcgtggct gtacaccgcg tccctggcct gcaccacacc    1380
   tacgatgtcc tcttcctggg cactggtgac ggccggctcc acaaggcagt gagcgtgggc    1440
   ccccgggtgc acatcattga ggagctgcag atcttctcat cgggacagcc cgtgcagaat    1500
   ctgctcctgg acacccacag ggggctgctg tatgcggcct cacactcggg cgtagtccag    1560
   gtgcccatgg ccaactgcag cctgtaccgg agctgtgggg actgcctcct cgcccgggac    1620
   ccctactgtg cttggagcgg ctccagctgc aagcacgtca gcctctacca gcctcagctg    1680
   gccaccaggc cgtggatcca ggacatcgag ggagccagcg ccaaggacct ttgcagcgcg    1740
   tcttcggttg tgtccccgtc ttttgtacca acaggggaga agccatgtga gcaagtccag    1800
   ttccagccca acacagtgaa cactttggcc tgcccgctcc tctccaacct ggcgacccga    1860
   ctctggctac gcaacggggc ccccgtcaat gcctcggcct cctgccacgt gctacccact    1920
   ggggacctgc tgctggtggg cacccaacag ctgggggagt tccagtgctg gtcactagag    1980
   gagggcttcc agcagctggt agccagctac tgcccagagg tggtggagga cggggtggca    2040
   gaccaaacag atgagggtgg cagtgtaccc gtcattatca gcacatcgcg tgtgagtgca    2100
   ccagctggtg gcaaggccag ctggggtgca gacaggtcct actggaagga gttcctggtg    2160
   atgtgcacgc tctttgtgct ggccgtgctg ctcccagttt tattcttgct ctaccggcac    2220
   cggaacagca tgaaagtctt cctgaagcag ggggaatgtg ccagcgtgca ccccaagacc    2280
   tgccctgtgg tgctgccccc tgagacccgc ccactcaacg gcctagggcc ccctagcacc    2340
   ccactcgatc accgagggta ccagtccctg tcagacagcc ccccggggtc ccgagtcttc    2400
   actgagtcag agaagaggcc actcagcatc caagacagct tcgtggaggt atccccagtg    2460
   tgcccccggc cccgggtccg ccttggctcg gagatccgtg actctgtggt g             2511

<210> 12
<211> 3766
<212> DNA
<213> Homo sapiens

<400> 12
   gctctgccca agccgaggct gcggggccgg cgccggcggg aggactgcgg tgccccgcgg 60
   aggggctgag tttgccaggg cccacttgac cctgtttccc acctcccgcc ccccaggtcc 120
   ggaggcgggg gcccccgggg cgactcgggg gcggaccgcg gggcggagct gccgcccgtg 180
   agtccggccg agccacctga gcccgagccg cgggacaccg tcgctcctgc tctccgaatg 240
   ctgcgcaccg cgatgggcct gaggagctgg ctcgccgccc catggggcgc gctgccgcct 300
   cggccaccgc tgctgctgct cctgctgctg ctgctcctgc tgcagccgcc gcctccgacc 360
   tgggcgctca gcccccggat cagcctgcct ctgggctctg aagagcggcc attcctcaga 420
   ttcgaagctg aacacatctc caactacaca gcccttctgc tgagcaggga tggcaggacc 480
   ctgtacgtgg gtgctcgaga ggccctcttt gcactcagta gcaacctcag cttcctgcca 540
   ggcggggagt accaggagct gctttggggt gcagacgcag agaagaaaca gcagtgcagc 600
   ttcaagggca aggacccaca gcgcgactgt caaaactaca tcaagatcct cctgccgctc 660
   agcggcagtc acctgttcac ctgtggcaca gcagccttca gccccatgtg tacctacatc 720
   aacatggaga acttcaccct ggcaagggac gagaagggga atgtcctcct ggaagatggc 780
   aagggccgtt gtcccttcga cccgaatttc aagtccactg ccctggtggt tgatggcgag 840
   ctctacactg gaacagtcag cagcttccaa gggaatgacc cggccatctc gcggagccaa 900
   agccttcgcc ccaccaagac cgagagctcc ctcaactggc tgcaagaccc agcttttgtg 960
   gcctcagcct acattcctga gagcctgggc agcttgcaag gcgatgatga caagatctac 1020
   tttttcttca gcgagactgg ccaggaattt gagttctttg agaacaccat tgtgtcccgc 1080
   attgcccgca tctgcaaggg cgatgagggt ggagagcggg tgctacagca gcgctggacc 1140
   tccttcctca aggcccagct gctgtgctca cggcccgacg atggcttccc cttcaacgtg 1200
   ctgcaggatg tcttcacgct gagccccagc ccccaggact ggcgtgacac ccttttctat 1260
   ggggtcttca cttcccagtg gcacagggga actacagaag gctctgccgt ctgtgtcttc 1320
   acaatgaatg atgtgcagag agtcttcagc ggcctctaca aggaggtgaa ccgtgagaca 1380
   cagcagatgg tacaccgtga cccacccgtg cccacacccc ggcctggagc gtgcatcacc 1440
   aacagtgccc gggaaaggaa gatcaactca tccctgcagc tcccagaccg cgtgctgaac 1500
   tttctcaagg accacttcct gatggacggg caggtccgaa gccgcatgct gctgctgcag 1560
   ccccaggctc gctaccagcg cgtggctgta caccgcgtcc ctggcctgca ccacacctac 1620
   gatgtcctct tcctgggcac tggtgacggc cggctccaca aggcagtgag cgtgggcccc 1680
   cgggtgcaca tcattgagga gctgcagatc ttctcatcgg gacagcccgt gcagaatctg 1740
   ctcctggaca cccacagggg gctgctgtat gcggcctcac actcgggcgt agtccaggtg 1800
   cccatggcca actgcagcct gtaccggagc tgtggggact gcctcctcgc ccgggacccc 1860
   tactgtgctt ggagcggctc cagctgcaag cacgtcagcc tctaccagcc tcagctggcc 1920
   accaggccgt ggatccagga catcgaggga gccagcgcca aggacctttg cagcgcgtct 1980
   tcggttgtgt ccccgtcttt tgtaccaaca ggggagaagc catgtgagca agtccagttc 2040
   cagcccaaca cagtgaacac tttggcctgc ccgctcctct ccaacctggc gacccgactc 2100
   tggctacgca acggggcccc cgtcaatgcc tcggcctcct gccacgtgct acccactggg 2160
   gacctgctgc tggtgggcac ccaacagctg ggggagttcc agtgctggtc actagaggag 2220
   ggcttccagc agctggtagc cagctactgc ccagaggtgg tggaggacgg ggtggcagac 2280
   caaacagatg agggtggcag tgtacccgtc attatcagca catcgcgtgt gagtgcacca 2340
   gctggtggca aggccagctg gggtgcagac aggtcctact ggaaggagtt cctggtgatg 2400
   tgcacgctct ttgtgctggc cgtgctgctc ccagttttat tcttgctcta ccggcaccgg 2460
   aacagcatga aagtcttcct gaagcagggg gaatgtgcca gcgtgcaccc caagacctgc 2520
   cctgtggtgc tgccccctga gacccgccca ctcaacggcc tagggccccc tagcacccca 2580
   ctcgatcacc gagggtacca gtccctgtca gacagccccc cggggtcccg agtcttcact 2640
   gagtcagaga agaggccact cagcatccaa gacagcttcg tggaggtatc cccagtgtgc 2700
   ccccggcccc gggtccgcct tggctcggag atccgtgact ctgtggtgtg agagctgact 2760
   tccagaggac gctgccctgg cttcaggggc tgtgaatgct cggagagggt caactggacc 2820
   tcccctccgc tctgctcttc gtggaacacg accgtggtgc ccggcccttg ggagccttgg 2880
   ggccagctgg cctgctgctc tccagtcaag tagcgaagct cctaccaccc agacacccaa 2940
   acagccgtgg ccccagaggt cctggccaaa tatgggggcc tgcctaggtt ggtggaacag 3000
   tgctccttat gtaaactgag ccctttgttt aaaaaacaat tccaaatgtg aaactagaat 3060
   gagagggaag agatagcatg gcatgcagca cacacggctg ctccagttca tggcctccca 3120
   ggggtgctgg ggatgcatcc aaagtggttg tctgagacag agttggaaac cctcaccaac 3180
   tggcctcttc accttccaca ttatcccgct gccaccggct gccctgtctc actgcagatt 3240
   caggaccagc ttgggctgcg tgcgttctgc cttgccagtc agccgaggat gtagttgttg 3300
   ctgccgtcgt cccaccacct cagggaccag agggctaggt tggcactgcg gccctcacca 3360
   ggtcctgggc tcggacccaa ctcctggacc tttccagcct gtatcaggct gtggccacac 3420
   gagaggacag cgcgagctca ggagagattt cgtgacaatg tacgcctttc cctcagaatt 3480
   cagggaagag actgtcgcct gccttcctcc gttgttgcgt gagaacccgt gtgccccttc 3540
   ccaccatatc caccctcgct ccatctttga actcaaacac gaggaactaa ctgcaccctg 3600
   gtcctctccc cagtccccag ttcaccctcc atccctcacc ttcctccact ctaagggata 3660
   tcaacactgc ccagcacagg ggccctgaat ttatgtggtt tttatacatt ttttaataag 3720
   atgcacttta tgtcattttt taataaagtc tgaagaatta ctgttt 3766

<210> 13
<211> 20
<212> DNA
<213> Artificial Sequence

<220>
<223> Oligonucleotide

<400> 13

cagtgccaac ctagccctct 20

<210> 14
<211> 20
<212> DNA
<213> Artificial Sequence

<220>
<223> Oligonucleotide

<400> 14

tctcccgatc caaccgtgac 20

<210> 15
<211> 20
<212> DNA
<213> Artificial Sequence

<220>
<223> Oligonucleotide

<400> 15

caacaactac atcctcggct 20

<210> 16
<211> 20
<212> DNA
<213> Artificial Sequence

<220>
<223> Oligonucleotide

<400> 16

tcggctccta catcaacaac 20

<210> 17
<211> 24
<212> DNA
<213> Artificial Sequence

<220>
<223> Primer

<400> 17

cctcgcccgg gacccctact gtgc 24

<210> 18
<211> 27
<212> DNA
<213> Artificial Sequence

<220>
<223> Primer

<400> 18

cttggcgctg gctccctcga tgtcctg                                          27

<210> 19
<211> 28
<212> DNA
<213> Artificial Sequence

<220>
<223> Primer

<400> 19

aattgaattc atgctgcgca ccgcgatg                                         28

<210> 20
<211> 30
<212> DNA
<213> Artificial Sequence

<220>
<223> Primer

<400> 20

aagctctaga caccacagag tcacggatct                                       30

<210> 21
<211> 30
<212> DNA
<213> Artificial Sequence

<220>
<223> Primer

<400> 21

aagctctaga tcacaccaca gagtcacgga                                       30

<210> 22
<211> 12
<212> PRT
<213> Homo sapiens

<400> 22

Asn Ser Ala Arg Glu Arg Lys Ile Asn Ser Ser Cys
                5                   10

<210> 23
<211> 15
<212> PRT
<213> Homo sapiens

<400> 23

Ser Val Val Ser Pro Ser Phe Val Pro Thr Gly Glu Lys Pro Cys
                5                   10                  15

<210> 24
<211> 15
<212> PRT
<213> Homo sapiens

<400> 24

Pro Leu Asp His Arg Gly Tyr Gln Ser Leu Ser Asp Ser Pro Cys
                5                   10                  15

<210> 25
<211> 14
<212> PRT
<213> Homo sapiens

<400> 25

Ser Arg Val Phe Thr Glu Ser Glu Lys Arg Pro Leu Ser Cys
                5                   10

## Description

### TECHNICAL FIELD

The present invention relates to a novel protein, a polynucleotide encoding the protein, a process of producing the protein, a prophylactic/therapeutic agent or a diagnostic drug for cancer, an apoptosis promoter, screening of the prophylactic/therapeutic agent for a cancer or the apoptosis promotor, etc.

### BACKGROND ART

Recent advance in microarray/oligonucleotide array technology has enabled exhaustive analysis of gene expression. It is predicted that a cancer could also be assessed for its pathological conditions by microarray profiling data for the gene. Actually in leukemia, it is reportedly possible to classify leukemia by gene expression profiles. By clarifying the gene expression profile of each cancerous tissue and accumulating its classification, it is considered possible to predict response to a particular cancer therapy or discover a novel drug development target protein for a particular cancer. Specifically, where enhanced expression of a certain protein is observed in a certain cancer, it becomes possible to induce an anti-tumor activity in patients newly diagnosed to be antigen positive, by means of (i) reducing its expression level, (ii) suppressing its function, (iii) eliciting immune response of host to the protein, etc. At the same time, patients diagnosed to be antigen negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based drugs.

The Semaphorin family is a large protein family consisting of both secreted molecules and membrane-bound molecules and there are reportedly at least 19 genes in vertebrate and 3 genes in non-vertebrate (Cell, 97, 551-552, 1999).

It is known that the Semaphorin family is involved in a wide range of the neurogenetic process represented by neuronal axon guidance, synapse formation, etc. In recent years, involvement of the Semaphorin family in the immune system (Trends in Immunol., 22, 670-676, 2001) and in organogenesis/angiogenesis has becoming clear. It is reported that human-derived Semaphorin 3B and Semaphorin 3F belonging to the Semaphorin family are tumor suppressor genes (Proc. Natl. Acad. Sci. USA, 98, 13954-13959, 2001, Cancer Res., 62, 542-546, 2002, Cancer Res., 62, 2637-2643, 2002). It is also reported that Semaphorin 3C is overexpressed in human lung cancer tissues (J. Surg. Oncol., 72, 18-23, 1999, Proc. Natl. Acad. Sci. USA, 94, 14713-14718, 1997). It is reported that Semaphorin 3E is expressed in metastatic cells (Cancer Res., 58, 1238-1244, 1998).

Semaphorin 4B (hereinafter sometimes briefly referred to SEMA4B) having 41 % homology with Semaphorin 4D on an amino acid level is registered in GenBank as a putative gene from the genome sequence (GenBank Accession No. XM_044533). SEMA4B is reported as one of genes overexpressed under hypoxic conditions (WO 02/46465). It is further reported that several hundreds of base sequences including SEMA4B, etc. can be used for search of compounds for diagnosis and treatment of lung cancer, based on the gene chip analysis (WO 02/86443). It is reported that NOV7 having 93% homology with SEMA4B on an amino acid level is overexpressed in cancer (WO 02/06329).

A safe drug, which targets at a molecule specifically expressed in cancer cells to induce growth inhibition of cancer cells, has been earnestly desired.

### DISCLOSURE OF THE INVENTION

The present inventors made extensive studies to solve the problems described above and as a result, have found a novel gene, expression of which is markedly enhanced in lung cancer tissues and also found that antisense oligonucleotide for this gene promotes apoptosis of cancer cells. Based on the findings, the inventors have continued further studies and come to accomplish the present invention.

That is, the present invention provides the following features and so on.
(1) A protein comprising the same or substantially the same amino acid sequence as the amino acid sequence represented by SEQ ID NO: 4, SEQ ID NO: 7 or SEQ ID NO: 10, or a salt thereof.
(2) A protein consisting of the amino acid sequence represented by SEQ ID NO: 4, SEQ ID NO: 7 or SEQ ID NO: 10, or a salt thereof.
(3) A partial peptide of the protein according to (1), or a salt thereof.
(4) A polynucleotide comprising a polynucleotide encoding the protein according to (1), or a partial peptide thereof.
(5) The polynucleotide according to (4), which is a DNA.
(6) The polynucleotide according to (5), which contains a base sequence represented by SEQ ID NO: 5, SEQ ID NO: 8 or SEQ ID NO: 11.
(7) A polynucleotide consisting of a base sequence represented by SEQ ID NO: 5, SEQ ID NO: 8 or SEQ ID NO: 11.
(8) A recombinant vector comprising the polynucleotide according to (4).
(9) A transformant transformed by the recombinant vector according to (8).
(10) A method of manufacturing the protein according to (1), its partial peptide, or a salt thereof, which comprises culturing the transformant according to (9), and producing/accumulating the protein according to (1) or its partial peptide.
(11) A pharmaceutical comprising the protein according to (1), its partial peptide, or a salt thereof.
(12) A pharmaceutical comprising the polynucleotide according to (4).
(13) A diagnostic agent comprising the polynucleotide according to (4).
(14) An antibody to the protein according to (1), its partial peptide, or a salt thereof.
(15) A pharmaceutical comprising the antibody according to (14).
(16) A diagnostic agent comprising the antibody according to (14).
(17) A polynucleotide comprising the entire or part of a base sequence complementary or substantially complementary to the polynucleotide according to (4).
(18) A pharmaceutical comprising the polynucleotide according to (17).
(19) A method of quantifying the protein according to (1), which comprises using the antibody according to (14).
(20) A method for diagnosis of a disease associated with the protein according to (1) or with its function, which comprises using the quantifying method according to (19).
(21) A method of screening a compound or its salt that inhibits the expression of the protein according to (1), which comprises using the protein according to (1), the partial peptide, or a salt thereof.
(22) A kit for screening a compound or its salt that inhibits the expression of the protein according to (1), comprising the protein according to (1), the partial peptide, or a salt thereof.
(22a) A compound or its salt that inhibits the expression of the protein according to (1), which is obtained by using the screening method according to (21) or the screening kit according to (22).
(22b) A pharmaceutical comprising the compound or its salt according to (22a).
(23) A method of screening a compound or its salt that inhibits the expression of a gene for the protein according to (1), which comprises using the polynucleotide according to (4).
(24) A kit for screening a compound or its salt that inhibits the expression of a gene for the protein according to (1), comprising the polynucleotide according to (4).
(24a) A compound or its salt that inhibits the expression of the protein according to (1), which is obtained by using the screening method according to (23) or the screening kit according to (24).
(24b) A pharmaceutical comprising the compound or its salt according to (24a).
(25) The pharmaceutical according to (11), (12), (15) or (18), which is a prophylactic/therapeutic agent for a cancer.
(25a) The pharmaceutical according to (25), wherein said cancer is lung cancer, ovary cancer or pancreatic cancer.
(25b) The pharmaceutical according to (22b) or (24b), which is a prophylactic/therapeutic agent for a cancer.
(26) The pharmaceutical according to (11), (12), (15) or (18), which is an apoptosis promoter (for cancer cells).
(26a) The pharmaceutical according to (22b) or (24b), which is an apoptosis promoter (for cancer cells).
(26b) The pharmaceutical according to (11), (12), (15), (18), (22b) or (24b), which is an agent for promoting growth inhibition of cancer cells.
(27) The diagnostic agent according to (13) or (16), which is a diagnostic agent for a cancer.
(28) An apoptosis promoter comprising a substance that inhibits the expression of the protein according to (1) or a partial peptide thereof, or the expression of a gene for said protein.
(29) An apoptosis promoter comprising an antibody to a protein comprising the same or substantially the same amino acid sequence as the amino acid sequence represented by SEQ ID NO: 1, its partial peptide, or a salt thereof.
(30) A prophylactic/therapeutic agent for a cancer, comprising an antibody to a protein comprising the same or substantially the same amino acid sequence as the amino acid sequence represented by SEQ ID NO: 1, its partial peptide, or a salt thereof.
(30a) The prophylactic/therapeutic agent according to (30), wherein said cancer is lung cancer, ovary cancer or pancreatic cancer.
(30b) An agent for promoting growth inhibition of cancer cells, comprising an antibody to a protein comprising the same or substantially the same amino acid sequence as the amino acid sequence represented by SEQ ID NO: 1, its partial peptide, or a salt thereof.
(31) A polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or a partial peptide thereof.
(32) A pharmaceutical comprising the polynucleotide according to (31).
(33) The pharmaceutical according to (32), which is an apoptosis promoter.
(33a) The pharmaceutical according to (32), which is an agent for promoting growth inhibition of cancer cells.
(34) A method of screening an apoptosis promoter, which comprises using a polynucleotide encoding a protein comprising the same or substantially the same amino acid sequence as the amino acid sequence represented by SEQ ID NO: 1, or a partial peptide thereof.
(35) A kit for screening an apoptosis promoter, comprising a polynucleotide encoding a protein comprising the same or substantially the same amino acid sequence as the amino acid sequence represented by SEQ ID NO: 1, or a partial peptide thereof.
(35a) An apoptosis promoter, which is obtainable by using the screening method according to (34) or the screening kit according to (35).
(36) An apoptosis promoter, comprising a substance that inhibits the expression of a protein comprising the same or substantially the same amino acid sequence as the amino acid sequence represented by SEQ ID NO: 1, or its partial peptide, or the expression of a gene for said protein.
(36a) An agent for promoting growth inhibition of cancer cells, comprising a substance that inhibits the expression of a gene for a protein comprising the same or substantially the same amino acid sequence as the amino acid sequence represented by SEQ ID NO: 1, or its partial peptide.
(37) A method of preventing/treating a cancer, which comprises administering to a mammal an effective dose of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof.
(38) A method of promoting apoptosis of cancer cells, which comprises administering to a mammal an effective dose of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof.
(39) A method of preventing/treating a cancer, which comprises inhibiting the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, or inhibiting the expression of a gene for said protein or its partial peptide.
(40) A method of promoting apoptosis of cancer cells, which comprises inhibiting the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, or inhibiting the expression of a gene for said protein or its partial peptide.
(41) Use of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof, to manufacture a prophylactic/therapeutic agent for a cancer.
(42) Use of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof, to manufacture an apoptosis promoter for cancer cells.

### BEST MODE FOR CARRYING OUT THE INVENTION

The protein comprising the same or substantially the same amino acid sequence as the amino acid sequence represented by SEQ ID NO: 1, SEQ ID NO: 4, SEQ ID NO: 7 or SEQ ID NO: 10 used in the present invention (hereinafter these proteins are briefly referred to as the protein of the present invention or sometimes as the protein used in the present invention) may be any protein derived from any cells of human and warm-blooded animals (e.g., guinea pig, rat, mouse, fowl, rabbit, swine, sheep, bovine, monkey, etc.) such as hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.; or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence comprising substantially the same amino acid sequence as that represented by SEQ ID NO: 1 includes amino acid sequences having at least about 95% homology, preferably at least about 98% homology, and more preferably at least about 99% homology, to the amino acid sequence shown by SEQ ID NO: 1; and so on.

Preferred examples of the protein comprising substantially the same amino acid sequence as the amino acid sequence represented by SEQ ID NO: 1 include proteins comprising substantially the same amino acid sequence as the amino acid sequence represented by SEQ ID NO: 1 and having an activity substantially equivalent to that of the protein containing the amino acid sequence represented by SEQ ID NO: 1, etc.

The amino acid sequence comprising substantially the same amino acid sequence as that represented by SEQ ID NO: 4 includes amino acid sequences having at least about 99.9% homology to the amino acid sequence shown by SEQ ID NO: 4; etc.

Preferred examples of the protein comprising substantially the same amino acid sequence as the amino acid sequence represented by SEQ ID NO: 4 include proteins comprising substantially the same amino acid sequence as the amino acid sequence represented by SEQ ID NO: 4 and having an activity substantially equivalent to that of the protein comprising the amino acid sequence represented by SEQ ID NO: 4, etc.

The amino acid sequence comprising substantially the same amino acid sequence as that represented by SEQ ID NO: 7 includes amino acid sequences having at least about 99.9% homology, to the amino acid sequence shown by SEQ ID NO: 7; etc.

Preferred examples of the protein comprising substantially the same amino acid sequence as the amino acid sequence represented by SEQ ID NO: 7 include proteins comprising substantially the same amino acid sequence as the amino acid sequence represented by SEQ ID NO: 7 and having an activity substantially equivalent to that of the protein comprising the amino acid sequence represented by SEQ ID NO: 7, etc.

The amino acid sequence comprising substantially the same amino acid sequence as that represented by SEQ ID NO: 10 includes amino acid sequences having at least about 99.9% homology, to the amino acid sequence shown by SEQ ID NO: 10; etc.

Preferred examples of the protein comprising substantially the same amino acid sequence as the amino acid sequence represented by SEQ ID NO: 10 include proteins comprising substantially the same amino acid sequence as the amino acid sequence represented by SEQ ID NO: 10 and having an activity substantially equivalent to that of the protein containing the amino acid sequence represented by SEQ ID NO: 10, etc.

Homology of the amino acid sequences can be measured under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The substantially equivalent is used to mean that the property of these properties is equivalent in terms of quality (e.g., physiologically or pharmacologically). Thus, the activity of the protein of the present invention is preferably equivalent (e.g., about 0.01 to 100 times, preferably about 0.1 to 10 times, more preferably 0.5 to 2 times), but differences in degree such as a level of the activity, quantitative factors such as a molecular weight of the protein may be present and allowable.

Examples of the protein used in the present invention include so-called muteins such as proteins comprising (1) (i) the amino acid sequence represented by SEQ ID NO: 1, of which at least 1 or 2 (e.g., about 1 to about 50, preferably about 1 to about 30, more preferably about 1 to about 10 and most preferably several (1 to 5)) amino acids are deleted, (ii) the amino acid sequence represented by SEQ ID NO: 1, to which at least 1 or 2 (e.g., about 1 to about 50, preferably about 1 to about 30, more preferably about 1 to about 10 and most preferably several (1 to 5)) amino acids are added, (iii) the amino acid sequence represented by SEQ ID NO: 1, in which at least 1 or 2 (e.g., about 1 to about 50, preferably about 1 to about 30, more preferably about 1 to about 10 and most preferably several (1 to 5)) amino acids are inserted, (iv) the amino acid sequence represented by SEQ ID NO: 1, in which at least 1 or 2 (e.g., about 1 to about 50, preferably about 1 to about 30, more preferably about 1 to about 10 and most preferably several (1 to 5)) amino acids are substituted by other amino acids, or (v) a combination of these amino acid sequences; so-called muteins such as proteins comprising (2) (i) the amino acid sequence represented by SEQ ID NO: 4, SEQ ID NO: 7 or SEQ ID NO: 10, of which at least 1 or 2 (e.g., about 1 to about 50, preferably about 1 to about 30, more preferably about 1 to about 10 and most preferably several (1 to 5)) amino acids are deleted, (ii) the amino acid sequence represented by SEQ ID NO: 4, SEQ ID NO: 7 or SEQ ID NO: 10, to which at least 1 or 2 (e.g., about 1 to about 50, preferably about 1 to about 30, more preferably about 1 to about 10 and most preferably several (1 to 5)) amino acids are added, (iii) the amino acid sequence represented by SEQ ID NO: 4, SEQ ID NO: 7 or SEQ ID NO: 10, in which at least 1 or 2 (e.g., about 1 to about 50, preferably about 1 to about 30, more preferably about 1 to about 10 and most preferably several (1 to 5)) amino acids are inserted, (iv) the amino acid sequence represented by SEQ ID NO: 4, SEQ ID NO: 7 or SEQ ID NO: 10, in which at least 1 or 2 (e.g., about 1 to about 50, preferably about 1 to about 30, more preferably about 1 to about 10 and most preferably several (1 to 5)) amino acids are substituted by other amino acids, or (v) a combination of these amino acid sequences; and the like.

Where the amino acid sequence is inserted, deleted or substituted as described above, the position of its insertion, deletion or substitution is not particularly limited.

Throughout the specification, the proteins are represented in accordance with the conventional way of describing proteins, that is, the N-terminus (amino terminus) at the left hand and the C-terminus (carboxyl terminus) at the right hand. In the protein used in the present invention including the protein comprising the amino acid sequence represented by SEQ ID NO: 1, the C-terminus may be in any form of a carboxyl group (-COOH), a carboxylate (-COO⁻), an amide (-CONH₂) and an ester (-COOR).

Herein, examples of R in the ester group include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.or an α-naphthyl-C₁₋₂ alkyl group such as α-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein of the present invention. Examples of the ester group in this case may be the C-terminal esters described above, etc.

Furthermore, examples of the protein used in the present invention include variants wherein the amino group at the N-terminal amino acid residues (e.g., methionine residue) is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as so-called glycoproteins having sugar chains; etc.

Specific examples of the protein used in the present invention are a protein comprising the amino acid sequence represented by SEQ ID NO: 1, a protein comprising the amino acid sequence represented by SEQ ID NO: 4, a protein comprising the amino acid sequence represented by SEQ ID NO: 7, a protein comprising the amino acid sequence represented by SEQ ID NO: 10, a protein and the like.

The partial peptide of the protein used in the present invention may be any peptide as long as it is a partial peptide of the protein used in the present invention described above and preferably has the property equivalent to that of the protein used in the present invention described above.

For example, there are used peptides containing, e.g., at least 20, preferably at least 50, more preferably at least 70, much more preferably at least 100, and most preferably at least 200 amino acids in the constituent amino acid sequence of the protein used in the present invention, etc.

The partial peptide used in the present invention may be peptides containing the amino acid sequence, of which at least 1 or 2 (preferably about 1 to about 20, more preferably about 1 to about 10 and most preferably several (1 to 5)) amino acids may be deleted; peptides, to which at least 1 or 2 (preferably about 1 to about 20, more preferably about 1 to about 10 and most preferably several (1 to 5)) amino acids may be added; peptides, in which at least 1 or 2 (preferably about 1 to about 20, more preferably about 1 to about 10 and most preferably several (1 to 5)) amino acids may be inserted; or peptides, in which at least 1 or 2 (preferably about 1 to about 20, more preferably about 1 to about 10, much more preferably several and most preferably about 1 to about 5) amino acids may be substituted by other amino acids.

In the partial peptide used in the present invention, the C-terminus may be in any form of a carboxyl group (-COOH), a carboxylate (-COO⁻), an amide (-CONH₂) or an ester (-COOR).

Furthermore, the partial peptide used in the present invention includes variants having a carboxyl group (or a carboxylate) at a position other than the C-terminus, those wherein the amino group at the N-terminal amino acid residues (e.g., methionine residue) is protected with a protecting group; those wherein the N-terminal region is cleaved in vivo and the glutamyl group thus formed is pyroglutaminated; those wherein a substituent on the side chain of an amino acid in the molecule is protected with a suitable protecting group, or conjugated proteins such as so-called glycoproteins having sugar chains; etc., as in the protein used in the present invention described above.

The partial peptide used in the present invention may also be used as an antigen for producing antibodies.

As salts of the protein or partial peptide used in the present invention, salts with physiologically acceptable acids (e.g., inorganic acids or organic acids) or bases (e.g., alkali metal salts) may be employed, preferably in the form of physiologically acceptable acid addition salts. Examples of such salts include salts with inorganic acids (e.g., hydrochloric acid, phosphoric acid, hydrobromic acid and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide used in the present invention or salts thereof may be manufactured by publicly known methods used to purify a protein from human or warm-blooded animal cells or tissues described above. Alternatively, they may also be manufactured by culturing transformants containing DNAs encoding these proteins. Furthermore, they may also be manufactured by a modification of the methods for peptide synthesis, which will be later described.

Where these proteins are manufactured from human or mammalian tissues or cells, human or non-human mammalian tissues or cells are homogenized, extracted with an acid or the like, and the extract is purified and isolated by a combination of chromatography techniques such as reverse phase chromatography, ion exchange chromatography, and the like.

To synthesize the protein or partial peptide used in the present invention or its salts, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (linear, branched or cyclic alkyl esterification of, e.g., methyl, ethyl, propyl, butyl, t-butyl, cyclopentyl, cyclohexyl, cycloheptyl, cyclooctyl, 2-adamantyl, etc.), aralkyl esterification (e.g., benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group used as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately chosen from publicly known groups and publicly known means.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide used in the present invention or salts thereof can be manufactured by publicly known methods for peptide synthesis, or by cleaving the protein used in the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part. Where the product contains protecting groups, these protecting groups are removed to give the desired peptide. Publicly known methods for condensation and elimination of the protecting groups are described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide used in the present invention. When the partial peptide obtained by the above methods is in a free form, the partial peptide can be converted into an appropriate salt by a publicly known method or its modification; when the partial peptide is obtained in a salt form, it can be converted into a free form or other different salt form by a publicly known method or its modification.

The polynucleotide encoding the protein used in the present invention may be any polynucleotide so long as it contains the base sequence encoding the protein used in the present invention described above. Preferably, the polynucleotide is a DNA. The DNA may also be any one of genomic DNA, genomic DNA library, cDNA derived from the cells or tissues described above, cDNA library derived from the cells or tissues described above and synthetic DNA.

The vector used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Examples of the DNA encoding the protein used in the present invention may be any one of:
(i) a DNA comprising the base sequence represented by SEQ ID NO: 2, or a DNA comprising a base sequence hybridizable to the base sequence represented by SEQ ID NO: 2 under high stringent conditions and encoding a protein which has the properties of substantially the same nature as those of the protein comprising the amino acid sequence represented by SEQ ID NO: 1 described above,
(ii) a DNA comprising the base sequence represented by SEQ ID NO: 5, or a DNA comprising a base sequence hybridizable to the base sequence represented by SEQ ID NO: 5 under high stringent conditions and encoding a protein which has the properties of substantially the same nature as those of the protein comprising the amino acid sequence represented by SEQ ID NO: 4 described above,
(iii) a DNA comprising the base sequence represented by SEQ ID NO: 8, or a DNA comprising a base sequence hybridizable to the base sequence represented by SEQ ID NO: 8 under high stringent conditions and encoding a protein which has the properties of substantially the same nature as those of the protein comprising the amino acid sequence represented by SEQ ID NO: 7 described above,
(iv) a DNA comprising the base sequence represented by SEQ ID NO: 11, or a DNA comprising a base sequence hybridizable to the base sequence represented by SEQ ID NO: 11 under high stringent conditions and encoding a protein which has the properties of substantially the same nature as those of the protein comprising the amino acid sequence represented by SEQ ID NO: 10 described above,

As the DNA that is hybridizable to the base sequence represented by SEQ ID NO: 2 under high stringent conditions, there are employed, for example, DNAs comprising base sequences having at least about 95% homology, preferably at least about 98% homology, and more preferably at least about 99% homology, to the base sequence represented by SEQ ID NO: 2; and the like.

As the DNA that is hybridizable to the base sequence represented by SEQ ID NO: 5 under high stringent conditions, there are employed, for example, DNAs comprising base sequences having at least about 99.9% homology to the base sequence represented by SEQ ID NO: 5; and the like.

As the DNA that is hybridizable to the base sequence represented by SEQ ID NO: 8 under high stringent conditions, there are employed, for example, DNAs comprising base sequences having at least about 99.9% homology to the base sequence represented by SEQ ID NO: 8; and the like.

As the DNA that is hybridizable to the base sequence represented by SEQ ID NO: 11 under high stringent conditions, there are employed, for example, DNAs comprising base sequences having at least about 99.9% homology to the base sequence represented by SEQ ID NO: 11; and the like.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration at about 19 mM at a temperature of about 65°C are most preferred.

More specifically, there are employed: (i) a DNA comprising the base sequence represented by SEQ ID NO: 2, a DNA comprising the base sequence represented by SEQ ID NO: 3, etc. as the DNA encoding the protein comprising the amino acid sequence represented by SEQ ID NO: 1; (ii) a DNA comprising the base sequence represented by SEQ ID NO: 5, a DNA comprising the base sequence represented by SEQ ID NO: 6, etc. as the DNA encoding the protein comprising the amino acid sequence represented by SEQ ID NO: 4; (iii) a DNA comprising the base sequence represented by SEQ ID NO: 8, a DNA comprising the base sequence represented by SEQ ID NO: 9, etc. as the DNA encoding the protein comprising the amino acid sequence represented by SEQ ID NO: 7; (iv) a DNA comprising the base sequence represented by SEQ ID NO: 11, a DNA comprising the base sequence represented by SEQ ID NO: 12, etc. as the DNA encoding the protein comprising the amino acid sequence represented by SEQ ID NO: 10; and the like.

The polynucleotide (e.g., DNA) encoding the partial peptide used in the present invention may be any polynucleotide so long as it contains the base sequence encoding the partial peptide used in the present invention described above. The polynucleotide may also be any of genomic DNA, genomic DNA library, cDNA derived from the cells and tissues described above, cDNA library derived from the cells and tissues described above and synthetic DNA.

As the DNA encoding the partial peptide used in the present invention, there are employed, for example, a DNA comprising a part of the DNA having the base sequence represented by SEQ ID NO: 2, SEQ ID NO: 5, SEQ ID NO: 8 or SEQ ID NO: 11, or a DNA comprising a base sequence hybridizable to the base sequence represented by SEQ ID NO: 2, SEQ ID NO: 5, SEQ ID NO: 8 or SEQ ID NO: 11 under high stringent conditions and comprising a part of DNA encoding a protein having the activities of substantially the same nature as those of the protein of the present invention, and the like.

The DNA hybridizable to the base sequence represented by SEQ ID NO: 2, SEQ ID NO: 5, SEQ ID NO: 8 or SEQ ID NO: 11 indicates the same meaning as described above.

Methods for the hybridization and the high stringent conditions that can be used are the same as those described above.

For cloning of DNAs that completely encode the protein or partial peptide used in the present invention (hereinafter sometimes merely referred to as the protein of the present invention in the description of cloning of DNAs encoding the protein and partial peptide and their expression), the DNA can be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd (J. Sambrook et al., Cold Spring Harbor Lab. Press, 1989). Where the hybridization is carried out using commercially available library, the procedures may be conducted in accordance with the protocol described in the attached instructions.

Substitution of the base sequence of DNA can be effected by publicly known methods such as the ODA-LA PCR method, the Gapped duplex method, the Kunkel method, etc., or its modification, using PCR, a publicly known kit available as Mutan™-super Express Km (manufactured by Takara Shuzo Co., Ltd.) or Mutan™ -K (manufactured by Takara Shuzo Co., Ltd.), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, it is preferred to use CMV (cytomegalovirus) promoter, SRα promoter, etc. Where the host is bacteria of the genus Escherichia, preferred examples of the promoter include trp promoter, lac promoter, recA promoter, λP_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, selection can also be made on a thymidine free medium.

If necessary, a signal sequence that matches with a host is added to the N-terminus of the protein of the present invention. Examples of the signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include monkey cell COS-7, Vero, Chinese hamster cell CHO (hereinafter referred to as CHO cell), dhfr gene-deficient Chinese hamster cell CHO (hereinafter simply referred to as CHO (dhfr⁻) cell), mouse L cell, mouse AtT-20, mouse myeloma cell, mouse ATDC5 cell, rat GH3, human FL cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors containing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature), 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside of the transformant.

The protein of the present invention can be separated and purified from the culture described above by the following procedures.

When the protein of the present invention is extracted from the bacteria or cells, the bacteria or cell is collected after culturing by a publicly known method and suspended in an appropriate buffer. The bacteria or cell is then disrupted by publicly known methods such as ultrasonication, a treatment with lysozyme and/or freeze-thaw cycling, followed by centrifugation, filtration, etc to produce crude extract of the protein. Thus, the crude extract of the protein can be obtained. The buffer used for the procedures may contain a protein modifier such as urea or guanidine hydrochloride, or a surfactant such as Triton X-100™, etc. When the protein is secreted in the culture broth, the supernatant can be separated, after completion of the cultivation, from the bacteria or cell to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The antibodies to the protein or partial peptide used in the present invention, or its salts may be any of polyclonal and monoclonal antibodies, as long as they are capable of recognizing the protein or partial peptide used in the present invention, or its salts.

The antibodies to the protein or partial peptide used in the present invention, or its salts (hereinafter they are sometimes collectively referred to as the protein of the present invention in the description of the antibodies) can be produced by a publicly known method of producing an antibody or antiserum, using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every about 2 to about 6 weeks and about 2 to about 10 times in total. Examples of the applicable warm-blooded animals are monkeys, rabbits, dogs, guinea pigs, mice, rats, sheep, goats and fowl, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after 2 to 5 days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495, and (1975)]. Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at 20 to 40°C, preferably at 30 to 37°C for 1 to 10 minutes, an efficient cell fusion can be carried out.

Various methods can be used for screening of monoclonal antibody-producing hybridomas. Examples of such methods include a method which comprises adding the supernatant of a hybridoma to a solid phase (e.g., a microplate) adsorbed with the protein as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase, or the like.

The monoclonal antibody can be screened according to publicly known methods or their modifications. In general, the screening can be performed in a medium for animal cells supplemented with HAT (hypoxanthine, aminopterin and thymidine). Any screening and growth medium can be employed as far as the hybridoma can grow there. For example, RPMI 1640 medium containing 1 to 20%, preferably 10 to 20% fetal bovine serum, GIT medium (Wako Pure Chemical Industries, Ltd.) containing 1 to 10% fetal bovine serum, a serum free medium for cultivation of a hybridoma (SFM-101, Nissui Seiyaku Co., Ltd.) and the like, can be used for the screening and growth medium. The culture is carried out generally at 20 to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins [for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.]

### [Preparation of polyclonal antibody]

The polyclonal antibody of the present invention can be manufactured by publicly known methods or modifications thereof. For example, a warm-blooded animal is immunized with an immunogen (protein antigen) per se, or with a complex of immunogen and a carrier protein formed in a manner similar to the method described above for the manufacture of monoclonal antibodies. The product containing the antibody to the protein of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks and about 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The antisense polynucleotide having a complementary or substantially complementary base sequence to the base sequence of a polynucleotide encoding the protein or partial peptide used in the present invention (e.g., DNA (hereinafter these DNAs are sometimes collectively referred to as the DNA of the present invention in the description of antisense polynucleotide)) can be any antisense polynucleotide, so long as it possesses a base sequence complementary or substantially complementary to the base sequence of the polynucleotide (e.g., DNA) of the present invention and capable of suppressing the expression of said DNA, but antisense DNA is preferred.

The base sequence substantially complementary to the DNA of the present invention may include, for example, a base sequence having at least about 70% homology, preferably at least about 80% homology, more preferably at least about 90% homology and most preferably at least about 95% homology, to the entire base sequence or to its partial base sequence (i.e., complementary strand to the DNA of the present invention), and the like. Especially in the entire base sequence of the complementary strand to the DNA of the present invention, preferred are (a) an antisense polynucleotide having at least about 70% homology, preferably at least about 80% homology, more preferably at least about 90% homology and most preferably at least about 95% homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having at least about 70% homology, preferably at least about 80% homology, more preferably at least about 90% homology and most preferably at least about 95% homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to a base sequence of DNA containing the base sequence represented by SEQ ID NO: 2, SEQ ID NO: 3, SEQ ID NO: 5, SEQ ID NO: 6, SEQ ID NO: 8, SEQ ID NO: 9, SEQ ID NO: 11 or SEQ ID NO: 12, preferably an antisense polynucleotide containing the entire or part of a base sequence complementary to a base sequence of DNA containing the base sequence represented by SEQ ID NO: 2, SEQ ID NO: 3, SEQ ID NO: 5, SEQ ID NO: 6, SEQ ID NO: 8, SEQ ID NO: 9, SEQ ID NO: 11 or SEQ ID NO: 12, etc.

The antisense polynucleotide is generally constituted by bases of about 10 to about 40, preferably about 15 to about 30.

To prevent digestion with a hydrolase such as nuclease, etc., the phosphoric acid residue (phosphate) of each nucleotide that constitutes the antisense DNA may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 2. These antisense polynucleotides may be synthesized using a publicly known DNA synthesizer, etc.

According to the present invention, the antisense polynucleotide (nucleic acid) capable of inhibiting the replication or expression of a gene for the protein of the present invention can be designed and synthesized based on the base sequence information of cloned or identified protein-encoding DNA. Such a polynucleotide (nucleic acid) is hybridizable to RNA of a gene for the protein of the present invention to inhibit the synthesis or function of said RNA or is capable of modulating and/or controlling the expression of a gene for the protein of the present invention via interaction with RNA associated with the protein of the present invention. Polynucleotides complementary to the selected sequences of RNA associated with the protein of the present invention and polynucleotides specifically hybridizable to RNA associated with the protein of the present invention are useful in modulating/controlling the in vivo and in vitro expression of the protein gene of the present invention, and are useful for the treatment or diagnosis of diseases, etc. The term "corresponding" is used to mean homologous to or complementary to a particular sequence of the nucleotide including the gene, base sequence or nucleic acid. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense." Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid are sulfur and thiophosphate derivatives of nucleic acids and those resistant to degradation of polynucleoside amides or oligonucleoside amides, etc. The antisense polynucleotides of the present invention can be modified preferably based on the following design, that is, by increasing the intracellular stability of the antisense polynucleotide, more enhancing the cell permeability of the antisense polynucleotide, increasing the affinity of the nucleic acid to the targeted sense strand to a higher level, or minimizing the toxicity, if any, of the antisense polynucleotide. Most of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense polynucleotide can be examined using the transformant of the present invention, the gene expression system of the present invention in vivo and in vitro, or the translation system for the protein of the present invention in vivo and in vitro.

Hereinafter, the protein of the present invention, its partial peptides, or salts thereof (hereinafter sometimes merely referred to as the protein of the present invention), the DNA encoding the protein of the present invention or its partial peptides (hereinafter sometimes merely referred to as the DNA of the present invention), the antibodies to the protein of the present invention, its partial peptides, or salts thereof (hereinafter sometimes merely referred to as the antibody of the present invention) and the antisense polynucleotides to the DNA of the present invention (hereinafter sometimes merely referred to as the antisense polynucleotide of the present invention) are specifically described for their applications.

The protein of the present invention is increasingly expressed in cancer tissues and is thus available as a disease marker. That is, the protein is useful as a marker for early diagnosis in cancer tissues, for judgment of severity in conditions, or for predicted development of these diseases. Therefore, the pharmaceuticals comprising the antisense polynucleotide to the polynucleotide encoding the protein of the present invention, the compound or its salts that inhibits the activity of the protein of the present invention, the compound or its salts that inhibits the expression of a gene for the protein of the present invention, or the antibody to the protein of the present invention can be used as prophylactic/therapeutic agents for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), and as apoptosis promoters.

### (1) Screening of drug candidate compounds for disease

The protein of the present invention shows increased expression in cancer tissues. In addition, when the activity of the protein of the present invention is inhibited, cancer cells induce apoptosis. Thus, the compound or its salts that inhibit the activity of the protein of the present invention can be used as prophylactic/therapeutic agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), apoptosis promoters, etc.

Accordingly, the protein of the present invention is useful as a reagent for screening the compound or its salts that inhibit the activity of the protein of the present invention.

That is, the present invention provides a method of screening the compound or its salts that inhibit the activity of the protein of the present invention, which comprises using the protein of the present invention.

Specifically, there is employed the method of screening the compound or its salts that inhibit the activity of the protein of the present invention, which comprises comparing (i) the activity of a cell capable of producing the protein of the present invention with (ii) the activity of a mixture of the cell capable of producing the protein of the present invention and a test compound.

As the cells capable of producing the protein of the present invention, there are used, for example, the aforesaid host (transformant) transformed with a vector containing the DNA encoding the protein of the present invention. Preferably, animal cells such as COS7 cells, CHO cells, HEK293 cells, etc. are used as the host. For the screening, the transformant, in which the protein of the present invention has been expressed in the cells, e.g., by culturing through the procedure described above, is preferably employed. The procedure for incubating the cells capable of expressing the protein of the present invention is similar to the incubation procedure for the transformant of the present invention described above.

Examples of the test compound include peptides, proteins, non-peptide compounds, synthetic compounds, fermentation products, cell extracts, plant extracts, animal tissue extracts, etc.

For example, when a test compound inhibits the activity of the protein of the present invention in the case (ii) described above by at least about 20%, preferably at least 30% and more preferably at least about 50%, as compared to the case (i) above, the test compound can be selected as the compound that inhibits the activity of the protein of the present invention.

The compound having the activity of inhibiting the activity of the protein of the present invention is useful as a safe and low toxic pharmaceutical for suppressing the physiological activities of the protein of the present invention.

Furthermore, the gene for the protein of the present invention also shows an increased expression in cancer tissues. Accordingly, the compound or its salts that inhibit the expression of the gene for the protein of the present invention can also be used as a prophylactic/therapeutic agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), as an apoptosis promoter, etc..

Therefore, the polynucleotide (e.g., DNA) of the present invention is useful as a reagent for screening the compound or its salts inhibiting the expression of the gene for the protein of the present invention.

For the screening, there is a method of screening, which comprises comparing (iii) the case that a cell capable of producing the protein of the present invention is incubated and (iv) the case that a cell capable of producing the protein used in the present invention is incubated in the presence of a test compound.

In the screening method described above, the expression level of the gene described above (specifically, the level of the protein of the present invention or the level of mRNA encoding the said protein) is determined in the cases of (iii) and (iv), followed by comparison.

Examples of the test compound and the cells capable of producing the protein of the present invention are the same as described above.

The level of the protein can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The mRNA level can be determined by publicly known methods, e.g., in accordance with methods such as Northern hybridization using a nucleic acid containing the entire or a part of SEQ ID NO: 2, SEQ ID NO: 5, SEQ ID NO: 8 or SEQ ID NO: 11 as a probe, or PCR using a nucleic acid containing the entire or a part of SEQ ID NO: 2, SEQ ID NO: 5, SEQ ID NO: 8 or SEQ ID NO: 11 as a primer, or modifications thereof.

For example, when a test compound inhibits the expression of the gene in the case (iv) described above by at least about 20%, preferably at least 30% and more preferably at least about 50%, as compared to the case (iii) above, the test compound can be selected to be the compound capable of inhibiting the expression of the gene for the protein of the present invention.

The screening kit of the present invention comprises the protein used in the present invention, its partial peptide or salts thereof, or the cell capable of producing the protein used in the present invention, or its partial peptide.

The compound or its salts obtained by using the screening method or screening kit of the present invention is the test compound described above, e.g., a compound selected from peptides, proteins, non-peptide compounds, synthetic compounds, fermentation products, cell extracts, plant extracts, animal tissue extracts, plasma, etc., or its salt, which is a compound or its salt inhibiting the activity of the protein of the present invention, a compound or its salt inhibiting the expression of the gene for the protein of the present invention.

The salts of these compounds used are those given above as the salts of the protein of the present invention.

The compound or its salts that inhibit the activity of the protein of the present invention and the compound or its salts that inhibit the expression of the gene for the protein of the present invention are useful as pharmaceuticals, respectively, for example, as therapeutic/prophylactic agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or as apoptosis promoters, etc.

Where the compound or its salt obtained by using the screening method or screening kit of the present invention is used as the prophylactic/therapeutic agent described above, these compounds can be converted into pharmaceutical preparations in a conventional manner.

For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or excipient conventionally used in the field of pharmaceutical preparations. Examples of the vehicle or excipient for tablets are lactose, starch, sucrose, magnesium stearate, etc.

Examples of the composition for parenteral administration are injectable preparations, suppositories, etc. The injectable preparations may include dosage forms such as intravenous, subcutaneous, intracutaneous and intramuscular injections, drip infusions, intraarticular injections, etc. These injectable preparations may be prepared by methods publicly known. For example, the injectable preparations may be prepared by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the aforesaid antibody or its salt with conventional bases for suppositories.

Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; it is preferred that the aforesaid antibody is contained in about 5 to about 100 mg especially in the form of injection, and in 10 to 250 mg for the other forms.

Each composition described above may further contain other active components unless formulation causes any adverse interaction with the compound described above.

Since the pharmaceutical preparations thus obtained are safe and low toxic, they can be administered to human or warm-blooded animal (e.g., mouse, rat, rabbit, sheep, swine, bovine, horse, fowl, cat, dog, monkey, chimpanzee, etc.) orally or parenterally.

The dose of the above compound or its salts may vary depending upon its action, target disease, subject to be administered, route of administration, etc. For example, when the compound or its salt that inhibits the expression of the gene for the protein of the present invention is orally administered for the purpose of treating, e.g., breast cancer, the compound or its salt is generally administered to an adult (as 60 kg body weight) in a daily dose of about 0.1 to about 100 mg, preferably about 1.0 to about 50 mg and more preferably about 1.0 to about 20 mg. In parenteral administration, a single dose of the said compound or its salt may vary depending upon subject to be administered, target disease, etc. When the compound or its salt that inhibits the expression of the gene for the protein of the present invention is administered to an adult (as 60 kg body weight) in the form of an injectable preparation for the purpose of treating, e.g., breast cancer, it is advantageous to administer the compound or its salt at cancerous lesions by way of injection in a daily dose of about 0.01 to about 30 mg, preferably about 0.1 to about 20 mg, and more preferably about 0.1 to about 10 mg. For other animal species, the corresponding dose as converted per 60 kg weight can be administered.

### (2) Quantification for the protein of the present invention

The antibody of the present invention is capable of specifically recognizing the protein of the present invention and therefore can be used for quantification of the protein of the present invention in a test sample fluid, in particular, for quantification by sandwich immunoassay; etc.

That is, the present invention provides:
(i) a method of quantifying the protein of the present invention in a test sample fluid, which comprises competitively reacting the antibody of the present invention, a test sample fluid and a labeled form of the protein of the present invention, and measuring the ratio of the labeled form of the protein of the present invention bound to said antibody; and,
(ii) a method of quantifying the protein of the present invention in a test sample fluid, which comprises reacting a test sample fluid simultaneously or continuously with the antibody of the present invention immobilized on a carrier and another labeled antibody of the present invention, and then measuring the activity of the labeling agent on the insoluble carrier.

In the quantification method (ii) described above, it is preferred that one antibody is capable of recognizing the N-terminal region of the protein of the present invention, while another antibody is capable of reacting with the C-terminal region of the protein of the present invention.

The monoclonal antibody to the protein of the present invention (hereinafter sometimes referred to as the monoclonal antibody of the present invention) can be used to quantify the protein of the present invention. In addition, the protein can be detected by means of a tissue staining as well. For these purposes, the antibody molecule per se may be used or F (ab')₂, Fab' or Fab fractions of the antibody molecule may also be used.

The method of quantifying the protein of the present invention using the antibody of the present invention is not particularly limited. Any quantification method can be used, so long as the amount of antibody, antigen or antibody-antigen complex corresponding to the amount of antigen (e.g., the amount of the protein) in a test sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is particularly preferred to use the sandwich method described hereinafter.

Examples of the labeling agent used in the assay method using the labeling substance are radioisotopes, enzymes, fluorescent substances, luminescent substances, and the like. As the radioisotopes, there are used, e.g., [¹²⁵I], [¹³¹I], [³H], [¹⁴C], etc. The enzymes described above are preferably enzymes, which are stable and have a high specific activity, and include, e.g., β-galactosidase, β-glucosidase, alkaline phosphatase, peroxidase, malate dehydrogenase, etc. As the fluorescent substances, there are used, e.g., cyanine fluorescent dyes (e.g., Cy2, Cy3, Cy5, Cy5.5, Cy7 (manufactured by Amersham Biosciences Corp.), etc.), fluorescamine, fluorescein isothiocyanate, etc. As the luminescent substances described above there are used, e.g., luminol, a luminol derivative, luciferin, lucigenin, etc. Furthermore, the biotin-avidin system may be used as well for binding of an antibody or antigen to a labeling agent.

For immobilization of the antigen or antibody, physical adsorption may be used. Chemical binding techniques conventionally used for insolubilization or immobilization of proteins, enzymes, etc. may also be used. For carriers, there are used, e.g., insoluble polysaccharides such as agarose, dextran, cellulose, etc.; synthetic resin such as polystyrene, polyacrylamide, silicon, etc., and glass or the like.

In the sandwich method, the immobilized monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method of the present invention, antibodies that bind to different sites of the protein of the present invention are preferably used as the monoclonal antibodies of the present invention used for the primary and secondary reactions. That is, in the antibodies used for the primary and secondary reactions are, for example, when the antibody used in the secondary reaction recognizes the C-terminal region of the protein of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, the competitive method, the immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying each of these immunological methods to the quantification method of the present invention, any particular conditions or procedures are not required. Quantification system for the protein of the present invention or its salts is constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.

As described above, the protein of the present invention can be quantified with high sensitivity, using the antibody of the present invention.

Furthermore, when an increased level of the protein of the present invention is detected by quantifying the level of the protein of the present invention using the antibody of the present invention, it can be diagnosed that one suffers from cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.); or it is highly likely to suffer from these disease in the future.

Moreover, the antibody of the present invention can be used to detect the protein of the present invention, which is present in a test sample such as a body fluid, a tissue, etc. The antibody can also be used to prepare an antibody column for purification of the protein of the present invention, detect the protein of the present invention in each fraction upon purification, analyze the behavior of the protein of the present invention in the cells under investigation; etc.

### (3) Gene diagnostic agent

By using the DNA of the present invention, e.g., as a probe, an abnormality (gene abnormality) of the DNA or mRNA encoding the protein of the present invention or its partial peptide in human or warm-blooded animal (e.g., rat, mouse, guinea pig, rabbit, fowl, sheep, swine, bovine, horse, cat, dog, monkey, chimpanzee, etc.) can be detected. Therefore, the DNA is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression is detected by, e.g., Northern hybridization or DNA mutation is detected by the PCR-SSCP assay, it can be diagnosed that it is highly likely to suffer from a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.)

### (4) Pharmaceutical comprising the antisense polynucleotide

The antisense polynucleotide of the present invention that binds to the DNA of the present invention complementarily to inhibit expression of the DNA is low toxic and can suppress the functions or effects of the protein of the present invention or the DNA of the present invention in vivo. Thus, the antisense polynucleotide can be used as a prophylactic/therapeutic agent for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or as an apoptosis promoter, etc..

Where the antisense polynucleotide described above is used as the aforesaid prophylactic/therapeutic agent or as the promoter, it can be prepared into pharmaceutical preparations by publicly known methods, which are provided for administration.

For example, the antisense polynucleotide described above alone is administered directly, or after the antisense polynucleotide is inserted into an appropriate vector such as retrovirus vector, adenovirus vector, adenovirus-associated virus vector, etc., the antisense polynucleotide may be administered orally or parenterally to human or a mammal (e.g., rat, rabbit, sheep, swine, bovine, cat, dog, monkey, etc.) in a conventional manner. The antisense polynucleotide may also be administered as it stands, or may be prepared into pharmaceutical preparations together with a physiologically acceptable carrier to assist its uptake, which are then administered by gene gun or through a catheter such as a catheter with a hydrogel. Alternatively, the antisense polynucleotide may be prepared into an aerosol, which is topically administered into the trachea as an inhaler.

Further for the purposes of improving pharmacokinetics, prolonging a half-life and improving intracellular uptake efficiency, the antisense polynucleotide described above is prepared into pharmaceutical preparations (injectable preparations) alone or together with a carrier such as liposome, etc. and the preparations may be administered intravenously, subcutaneously, etc.

A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.

In addition, the antisense polynucleotide may also be used as an oligonucleotide probe for diagnosis to examine the presence of the DNA of the present invention in tissues or cells and states of its expression.

As the antisense polynucleotide described above can, the double-stranded RNA containing a part of RNA encoding the protein of the present invention, ribozyme containing a part of RNA encoding the protein of the present invention, etc. can also prevent expression of the gene of the present invention to suppress the in vivo function of the protein used in the present invention or the DNA used in the present invention and thus can be used as a prophylactic/therapeutic agent for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or as an apoptosis promoter, etc.

The double-stranded RNA can be designed based on a sequence of the polynucleotide of the present invention and manufactured by modifications of publicly known methods (e.g., Nature, 411, 494, 2001).

The ribozyme can be designed based on a sequence of the polynucleotide of the present invention and manufactured by modifications of publicly known methods (e.g., TRENDS in Molecular Medicine, 7, 221, 2001). For example, the ribozyme can be manufactured by ligating a publicly known ribozyme to a part of the RNA encoding the protein of the present invention. A part of the RNA encoding the protein of the present invention includes a portion proximal to a cleavage site on the RNA of the present invention, which may be cleaved by a publicly known ribozyme (RNA fragment).

Where the double-stranded RNA or ribozyme described above is used as the prophylactic/therapeutic agent described above, the double-stranded RNA or ribozyme is prepared into pharmaceutical preparations as in the antisense polynucleotide, and the preparations can be provided for administration.

### (5) Pharmaceutical comprising the antibody of the present invention

The antibody of the present invention has the activity of inducing apoptosis of cancer cells and hence, can be used as a prophylactic/therapeutic agent (e.g., vaccine, etc.) for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, a prophylactic/therapeutic agent for breast cancer, lung cancer, pancreatic cancer, etc.), as an apoptosis promoter, and the like.

Since the aforesaid prophylactic/therapeutic agent for diseases and promoters comprising the antibody of the present invention are safe and low toxic, they can be administered to human or a mammal (e.g., rat, rabbit, sheep, swine, bovine, cat, dog, monkey, etc.) orally or parenterally (e.g., intravascularly, subcutaneously, etc.) either as liquid preparations as they are or as pharmaceutical compositions of adequate dosage form. Preferably, they can be administered in the form of vaccine in a conventional manner.

The antibody of the present invention may be administered in itself or as an appropriate pharmaceutical composition. The pharmaceutical composition used for the administration may contain the antibody of the present invention and its salt, a pharmacologically acceptable carrier, a diluent or an excipient. Such a composition is provided in the form of pharmaceutical preparations suitable for oral or parenteral administration.

Examples of the composition for parenteral administration are injectable preparations, suppositories, vaccine, etc. The injectable preparations may include dosage forms such as intravenous, subcutaneous, intracutaneous and intramuscular injections, drip infusions, etc. These injectable preparations may be prepared by methods publicly known. The injectable preparations may be prepared, e.g., by dissolving, suspending or emulsifying the antibody of the present invention or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mols) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the aforesaid antibody or its salt with conventional bases for suppositories.

Examples of the composition for oral administration include solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Advantageously, the pharmaceutical compositions for oral or parenteral use described above are prepared into pharmaceutical preparations in a unit dose suited to fit a dose of the active ingredients. Such unit dose preparations include, for example, tablets, pills, capsules, injections (ampoules), suppositories, etc. The amount of the aforesaid antibody contained is generally about 5 to 500 mg per dosage unit form; especially in the form of injection, it is preferred that the aforesaid antibody is contained in about 5 to 100 mg and in about 10 to 250 mg for the other forms.

The dose of the aforesaid prophylactic/therapeutic agent or regulator comprising the antibody of the present invention may vary depending upon subject to be administered, target disease, conditions, route of administration, etc. For example, when it is used for the purpose of treating/preventing, e.g., breast cancer in an adult, it is advantageous to intravenously administer the antibody of the present invention in a single dose of about 0.01 to about 20 mg/kg body weight, preferably about 0.1 to about 10 mg/kg body weight and more preferably about 0.1 to about 5 mg/kg body weight in approximately 1 to 5 times a day, preferably in approximately 1 to 3 times a day. In other parenteral administration and oral administration, the prophylactic/therapeutic agent or regulator can be administered in a dose corresponding to the dose given above. When the condition is especially severe, the dose may be increased according to the condition.

The antibody of the present invention may be administered in itself or in the form of an appropriate pharmaceutical composition. The pharmaceutical composition used for the administration may contain a pharmacologically acceptable carrier with the aforesaid antibody or its salts, a diluent or excipient. Such a composition is provided in the form of pharmaceutical preparations suitable for oral or parenteral administration (e:g., intravascular injection, subcutaneous injection, etc.).

Each composition described above may further contain other active components unless formulation causes any adverse interaction with the antibody described above.

Moreover, the antibody of the present invention may be used in combination with other drugs, for example, alkylating agents (e.g., cyclophosphamide, iphosphamide, etc.), antimetabolites (e.g., methotrexate, 5-fluorouracil, etc.), anticancer antibiotics (e.g., mitomycin, adriamycin, etc.), plant-derived antitumor agents (e.g., vincristine, vinblastine, vindesine, taxol, etc.), cisplatin, carboplatin, etopoxide, etc. The antibody of the present invention and the drugs described above may be administered simultaneously or at staggered times to the patient.

### (6) Pharmaceutical comprising the protein of the present invention

Since the protein of the present invention is overexpressed in cancers, the protein of the present invention can be used as a cancer vaccine to activate the immune system in patients with cancer.

For example, the so-called adoptive immunotherapy, which involves culturing potent antigen presenting cells (e.g., dendritic cells) in the presence of the protein of the present invention to engulf the protein and putting the cells back into the body, can preferably be used. The dendritic cells, returned back into the body, can induce and activate cytotoxic T cells specific to a cancer antigen whereby to kill cancer cells.

The protein of the present invention can also be administered to a mammal (e.g. human, monkey, mouse, rat, rabbit, swine) safely as a vaccine preparation to prevent or treat a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.)

The vaccine preparation usually contains the protein of the present invention and a physiologically acceptable carrier. Such a carrier includes a liquid carrier such as water, saline (including physiological saline), buffer (e.g., phosphate buffer), an alcohol (e.g., ethanol), etc.

The vaccine preparation can be prepared according to a conventional method of manufacturing a vaccine preparation.

In general, the protein of the present invention is dissolved or suspended in a physiologically acceptable carrier. Alternatively, the protein of the present invention and the physiologically acceptable carrier may be separately prepared and then mixed at use.

The vaccine preparation may be further formulated with, for example, an adjuvant (e.g., aluminum hydroxide gel, serum albumin, etc.), a preservative (e.g., thimerosal, etc.), a soothing agent (e.g., glucose, benzyl alcohol, etc.), in addition to the protein of the present invention and the physiologically acceptable carrier. Furthermore, the vaccine preparation may also be formulated with, for example, a cytokine (e.g., an interleukin such as interleukin-2, an interferon such as interferon-y) to enhance the production of the antibody to the protein of the present invention.

When used as a vaccine preparation, the protein of the present invention may be used in its active form, or may be denatured to enhance the antigenicity. The protein of the present invention can be denatured usually by heating or treating with a protein-denaturing agent (e.g., formalin, guanidine hydrochloride and urea).

The thus obtained vaccine preparation is low toxic and may usually be administered in an injectable form, e.g., subcutaneously, intracutaneously, intramuscularly, or topically into or near a mass of cancer cells.

The dose of the protein of the present invention varies depending on a target disease, a subject to be administered, a route for administration, etc. For example, for subcutaneous administration of the protein of the present invention to an adult cancer patient (60 kg body weight) in an injectable form, the single dose is normally about 0.1 mg to about 300 mg, preferably about 100 mg to about 300 mg. The administration of the vaccine preparation may be carried out once, or 2 to 4 times in total approximately in every 2 weeks to 6 months to increase the production of the antibody.

### (7) DNA transgenic animal

The present invention provides a non-human mammal bearing a DNA encoding the protein of the present invention, which is exogenous (hereinafter abbreviated as the exogenous DNA of the present invention) or its variant DNA (sometimes simply referred to as the exogenous variant DNA of the present invention).

That is, the present invention provides:
(1) A non-human mammal bearing the exogenous DNA of the present invention or its variant DNA;
(2) The mammal according to (1), wherein the non-human mammal is a rodent;
(3) The mammal according to (2), wherein the rodent is mouse or rat; and,
(4) A recombinant vector containing the exogenous DNA of the present invention or its variant DNA and capable of expressing in a mammal; etc.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle from a standpoint of creating model animals for human disease.

"Mammals" in a recombinant vector that can be expressed in the mammals include the aforesaid non-human mammals, human, etc.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses the abnormal protein of the present invention and exemplified by the DNA that expresses a protein for suppressing the function of the normal protein of the present invention.

The exogenous DNA of the present invention may be any one of those derived from a mammal of the same species as, or a different species from, the mammal as the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression described above include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K 14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for the normal protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal protein obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transfected can be passaged as the DNA-bearing animal under ordinary rearing environment, by confirming that the exogenous DNA is stably retained by crossing.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

It is possible to obtain homozygous animals having the transfected DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.

Furthermore, since a mammal transfected with the exogenous normal DNA of the present invention exhibits an increasing symptom of the protein of the present invention liberated, the animal is usable for screening test of prophylactic/therapeutic agents for diseases associated with the protein of the present invention, for example, the prophylactic/therapeutic agent for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the animal after DNA transfection means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the protein of the present invention and the pathological mechanism of the disease and to investigate how to treat the disease.

More specifically, the transgenic animal expressing the abnormal DNA of the present invention at a high level is expected to serve as an experimental model to elucidate the mechanism of the functional inhibition (dominant negative effect) of a normal protein by the abnormal protein of the present invention in the function inactive type inadaptability of the protein of the present invention.

Since a mammal bearing the abnormal exogenous DNA of the present invention shows an increased symptom of the protein of the present invention liberated, the animal is also expected to serve for screening test of prophylactic/therapeutic agents for the function inactive type inadaptability of the protein of the present invention, e.g., prophylactic/therapeutic agents for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

Other potential applications of two kinds of the DNA transgenic animals of the present invention described above further include:
(i) Use as a cell source for tissue culture;
(ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
(iii) Research on the function of cells derived from tissues that are usually cultured only with difficulty, using cells in tissues bearing the DNA cultured by a standard tissue culture technique;
(iv) Screening a drug that enhances the functions of cells using the cells described in (iii) above; and,
(v) Isolation and purification of the variant protein of the present invention and preparation of an antibody thereto.

Furthermore, clinical conditions of a disease associated wit the protein of the present invention, including the function inactive type inadaptability to the protein of the present invention can be determined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the transgenic animal can serve to identify cells capable of producing the protein of the present invention, and to study in association with apoptosis, differentiation or propagation or on the mechanism of signal transduction in these properties to inspect any abnormality therein. Thus, the transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) A non-human mammal embryonic stem cell in which the DNA of the present invention is inactivated;
(2) The embryonic stem cell according to (1), wherein the DNA is inactivated by introducing a reporter gene (e.g., β-galactosidase gene derived from *Escherichia coli);*
(3) The embryonic stem cell according to (1), which is resistant to neomycin;
(4) The embryonic stem cell according to (1), wherein the non-human mammal is a rodent;
(5) The embryonic stem cell according to (4), wherein the rodent is mouse;
(6) A non-human mammal deficient in expressing the DNA of the present invention, wherein the DNA is inactivated;
(7) The non-human mammal according to (6), wherein the DNA is inactivated by inserting a reporter gene (e.g., β-galactosidase derived from *Escherichia coli)* therein and the reporter gene is capable of being expressed under control of a promoter for the DNA of the present invention;
(8) The non-human mammal according to (6), which is a rodent;
(9) The non-human mammal according to (8), wherein the rodent is mouse; and,
(10) A method of screening a compound that promotes or inhibits (preferably inhibits) the promoter activity to the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used; alternatively, embryos are collected at the 8-cell stage, cultured until the blastocyte stage, and used, whereby a large number of early stage embryos can be efficiently obtained.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1 % trypsin/l mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the function of the protein of the present invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples given above apply.

With respect to the non-human mammal deficient in expression of the DNA of the present invention, the DNA of the present invention can be knockout by transfecting a targeting vector, prepared as described above, to mouse embryonic stem cells or mouse oocytes, and conducting homologous recombination in which a targeting vector DNA sequence, wherein the DNA of the present invention is inactivated by the transfection, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expression of the DNA of the present invention.

Since the non-human mammal deficient in expression of the DNA of the present invention lacks various biological activities derived from the protein of the present invention, such an animal can be a disease model suspected of inactivated biological activities of the protein of the present invention and thus, offers an effective study to investigate the causes for and therapy for these diseases.

### (8a) Method of screening the compound having a therapeutic/prophylactic effect on diseases caused by deficiency, damages, etc. of the DNA of the present invention

The non-human mammal deficient in expression of the DNA of the present invention can be employed for screening the compound having a therapeutic/prophylactic effect on diseases caused by deficiency, damages, etc. of the DNA of the present invention.

That is, the present invention provides a method of screening the compound having a therapeutic/prophylactic effect on diseases, e.g., cancer, caused by deficiency, damages, etc. of the DNA of the present invention, which comprises administering a test compound to a non-human mammal deficient in expression of the DNA of the present invention and, observing and determining a change occurred in the animal.

As the non-human mammal deficient in expression of the DNA of the present invention, which can be employed for the screening method, the same examples as described above apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the administration route, property of the test compound, etc.

For screening of the compound having a therapeutic/prophylactic effect on a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), a test compound is administered to the non-human mammal deficient in expression of the DNA of the present invention. Differences in incidence of cancer or differences in degree of healing from the group administered with no test compound are observed in the tissues described above with passage of time.

In the screening method, when a test compound is administered to a test animal and the disease conditions of the test animal are improved by at least about 10%, preferably at least about 30% and more preferably at least about 50%, the test compound can be selected as the compound having the therapeutic/prophylactic effect on the diseases described above.

The compound obtained using the above screening method is a compound selected from the test compounds described above and exhibits a therapeutic/prophylactic effect on diseases caused by deficiencies, damages, etc. of the protein of the present invention. Therefore, the compound can be employed as a safe and low toxic drug for the prevention/treatment of the diseases. Furthermore, compounds derived from the compound obtained by the screening described above may also be used as well.

The compound obtained by the screening method above may form salts, and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A pharmaceutical comprising the compound obtained by the above screening method or salts thereof can be manufactured in a manner similar to the method for preparing the pharmaceutical comprising the protein of the present invention described hereinabove.

Since the pharmaceutical preparation thus obtained is safe and low toxic, it can be administered to human or a mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered, the compound is administered to the adult patient with breast cancer (as 60 kg body weight) generally in a dose of about 0.1 to 100 mg, preferably about 1.0 to 50 mg and more preferably about 1.0 to 20 mg. In parenteral administration, a single dose of the compound may vary depending upon subject to be administered, target disease, etc. When the compound is administered to the adult patient with breast cancer (as 60 kg body weight) in the form of an injectable preparation, it is advantageous to administer the compound in a single dose of about 0.01 to about 30 mg, preferably about 0.1 to about 20 mg and more preferably about 0.1 to about 10 mg a day. For other animal species, the corresponding dose as converted per 60 kg weight can be administered.

### (8b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds described above.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and compounds that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts inhibiting the promoter activity to the DNA of the present invention can inhibit the expression of the protein of the present invention to inhibit the functions of the protein. Thus, the compound or its salt is useful as a prophylactic/therapeutic agent for a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

In addition, compounds derived from the compound obtained by the screening described above may also be used as well.

A pharmaceutical comprising the compound obtained by the above screening method or salts thereof can be manufactured in a manner similar to the method for preparing the pharmaceutical comprising the protein of the present invention described above.

Since the pharmaceutical preparation thus obtained is safe and low toxic, it can be administered to human or a mammal (e.g., rat, mouse, guinea pig, rabbit, sheep, swine, bovine, horse, cat, dog, monkey, etc.).

A dose of the compound or salts thereof may vary depending on target disease, subject to be administered, route for administration, etc.; when the compound that inhibits the promoter activity to the DNA of the present invention is orally administered, the compound is administered to the adult patient with breast cancer (as 60 kg body weight) normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the adult patient with breast cancer (as 60 kg body weight) in the form of injectable preparation, it is advantageous to administer the compound intravenously to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As such, the non-human mammal deficient in expression of the DNA of the present invention is extremely useful for screening the compound or its salt that promotes or inhibits the promoter activity to the DNA of the present invention and, can greatly contribute to elucidation of causes for various diseases suspected of deficiency in expression of the DNA of the present invention and for the development of prophylactic/therapeutic agents for these diseases.

In addition, a so-called transgenic animal (gene transferred animal) can be prepared by using a DNA containing the promoter region of the protein of the present invention, ligating genes encoding various proteins at the downstream and injecting the same into oocyte of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA :: deoxyribonucleic acid
- cDNA :: complementary deoxyribonucleic acid
- A: : adenine
- T: : thymine
- G: : guanine
- C: : cytosine
- RNA: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP :: adenosine triphosphate
- EDTA :: ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: histidine
- Phe: : phenylalanine
- Tyr: : tyrosine
- Trp: : tryptophan
- Pro: : proline
- Asn: : asparagine
- Gln: glutamine
- pGlu: : pyroglutamic acid
- Sec: : selenocysteine

Substituents, protecting groups and reagents generally used in this specification are presented as the codes below.
- Me: : methyl group
- Et: : ethyl group
- Bu: : butyl group
- Ph: : phenyl group
- TC: : thiazolidine-4(R)-carboxamido group
- Tos: : p-toluenesulfonyl
- CHO :: formyl
- Bzl: : benzyl
- Cl₂-Bzl :: 2,6-dichlorobenzyl
- Bom :: benzyloxymethyl
- Z: : benzyloxycarbonyl
- Cl-Z: :2-chlorobenzyloxycarbonyl
- Br-Z: : 2-bromobenzyl oxycarbonyl
- Boc: : t-butoxycarbonyl
- DNP :: dinitrophenol
- Trt: : trityl
- Bum :: t-butoxymethyl
- Fmoc :: N-9-fluorenyl methoxycarbonyl
- HOBt :: :1-hydroxybenztriazole
- HOOBt :: 3,4-dihydro-3-hydroxy-4-oxo-1,2,3-benzotriazine
- HONB :: :1-hydroxy-5-norbornene-2,3-dicarboxyimide
- DCC :: N,N'-dicyclohexylcarbodiimide

The sequence identification numbers in the sequence listing of the specification indicate the following sequences.

### [SEQ ID NO: 1]

This shows the amino acid sequence of SEMA4B.

### [SEQ ID NO: 2]

This shows the base sequence of DNA encoding SEMA4B having the amino acid sequence represented by SEQ ID NO: 1.

### [SEQ ID NO: 3]

This shows the base sequence of DNA containing the full-length gene encoding SEMA4B.

### [SEQ ID NO: 4]

This shows the amino acid sequence of SEMA4B-M1.

### [SEQ ID NO: 5]

This shows the base sequence of DNA encoding SEMA4B-M1 having the amino acid sequence represented by SEQ ID NO: 4.

### [SEQ ID NO: 6]

This shows the base sequence of DNA containing the full-length gene encoding SEMA4B-M1.

### [SEQ ID NO: 7]

This shows the amino acid sequence of SEMA4B-M2.

### [SEQ ID NO: 8]

This shows the base sequence of DNA encoding SEMA4B-M2 having the amino acid sequence represented by SEQ ID NO: 7.

### [SEQ ID NO: 9]

This shows the base sequence of DNA containing the full-length gene encoding SEMA4B-M2.

### [SEQ ID NO: 10]

This shows the amino acid sequence of SEMA4B-M3.

### [SEQ ID NO: 11]

This shows the base sequence of DNA encoding SEMA4B-M3 having the amino acid sequence represented by SEQ ID NO: 10.

### [SEQ ID NO: 12]

This shows the base sequence of DNA containing the full-length gene encoding SEMA4B-M3.

### [SEQ ID NO: 13]

This shows the base sequence of antisense oligonucleotide used in EXAMPLES 2, 3, 15 and 16.

### [SEQ ID NO: 14]

This shows the base sequence of oligonucleotide used in EXAMPLES 2, 3, 15 and 16.

### [SEQ ID NO: 15]

This shows the base sequence of antisense oligonucleotide used in EXAMPLE 3.

### [SEQ ID NO: 16]

This shows the base sequence of oligonucleotide used in EXAMPLE 3.

### [SEQ ID NO: 17]

This shows the base sequence of primer used in EXAMPLE 3.

### [SEQ ID NO: 18]

This shows the base sequence of primer used in EXAMPLE 3.

### [SEQ ID NO: 19]

This shows the base sequence of primer used in EXAMPLES 4, 6 and 7.

### [SEQ ID NO: 20]

This shows the base sequence of primer used in EXAMPLES 4 and 7.

### [SEQ ID NO: 21]

This shows the base sequence of primer used in EXAMPLE 6.

### [SEQ ID NO: 22]

This shows the base sequence of Peptide 1 used in EXAMPLE 8.

### [SEQ ID NO: 23]

This shows the base sequence of Peptide 2 used in EXAMPLE 8

### [SEQ ID NO: 24]

This shows the base sequence of Peptide 3 used in EXAMPLE 8

### [SEQ ID NO: 25]

This shows the base sequence of Peptide 4 used in EXAMPLE 8

The transformant, Escherichia coli TOP10/SEMA4B-M1/pCR4-TOPO obtained in EXAMPLE 4 later described has been on deposit since March 4, 2003 under the Accession Number FERM BP-8316 at the National Institute of Advanced Industrial Science and Technology, International Patent Organism Depositary, located at Central 6, 1-1-1 Higashi, Tsukuba, Ibaraki, Japan (postal code 305-8566).

The transformant, Escherichia coli TOP10/SEMA4B-M2/pCR4-TOPO obtained in EXAMPLE 4 later described has been on deposit since March 4, 2003 under the Accession Number FERM BP-8317 at the National Institute of Advanced Industrial Science and Technology, International Patent Organism Depositary, located at Central 6, 1-1-1 Higashi, Tsukuba, Ibaraki, Japan (postal code 305-8566).

The transformant, Escherichia coli TOP10/SEMA4B-M3/pCR4-TOPO obtained in EXAMPLE 4 later described has been on deposit since March 4, 2003 under the Accession Number FERM BP-8318 at the National Institute of Advanced Industrial Science and Technology, International Patent Organism Depositary, located at Central 6, 1-1-1 Higashi, Tsukuba, Ibaraki, Japan (postal code 305-8566).

Hereinafter, the present invention will be described specifically with reference to EXAMPLES but is not deemed to be limited thereto.

### EXAMPLE 1

### Gene expression analysis

In order to clarify a group of genes with their expression enhanced specifically in lung cancer tissues, gene expression analysis was performed by oligonucleotide microarray (Human Genome U95A, U95B, U95C, U95D, U95E; Affymetrix) on total RNAs extracted from 4 lung cancer tissues and 5 normal lung tissues (TABLE 1) as samples. The experimental procedures were performed in accordance with the Affymetrix Corp. manual (Expression Analysis Technical Manual).

As a result, the overexpression of Semaphorin 4B (SEMA4B) gene and Semaphorin 4B-M1 (SEMA4B-M1), Semaphorin 4B-M2 (SEMA4B-M2) and Semaphorin 4B-M3 (SEMA4B-M3) genes later described in EXAMPLE 4 was detected in 3 lung cancer tissues (lot. 0011-192-01285, lot. 0011-192-01293 and lot. 0011-192-01297) (Table 2).

**[Table 1]**

| RNA-Extracted Tissue | Distribution Source |
|---|---|
| Lung cancer tissue (lot. 0009-192-00122) | BioClinical Partners, Inc. |
| Lung cancer tissue (lot. 0011-192-01285) | BioClinical Partners, Inc. |
| Lung cancer tissue (lot. 0011-192-01293) | BioClinical Partners, Inc. |
| Lung cancer tissue (lot. 0011-192-01297) | BioClinical Partners, Inc. |
| Normal lung tissue (lot. 0009-192-00150) | BioClinical Partners, Inc. |
| Normal lung tissue (lot. 0009-192-00168) | BioClinical Partners, Inc. |
| Normal lung tissue (lot. 0011-192-01283) | BioClinical Partners, Inc. |
| Normal lung tissue (lot. 0011-192-01285) | BioClinical Partners, Inc. |
| Normal lung tissue (lot. 0011-192-01297) | BioClinical Partners, Inc. |

**[Table 2]**

| Tissue | Gene Expression Level |
|---|---|
| Lung cancer tissue (lot. 0009-192-00122) | ND |
| Lung cancer tissue (lot. 0011-192-01285) | 10 |
| Lung cancer tissue (lot. 0011-192-01293) | 9.5 |
| Lung cancer tissue (lot. 0011-192-01297) | 1.9 |
| Normal lung tissue (lot. 0009-192-00150) | ND |
| Normal lung tissue (lot. 0009-192-00168) | ND |
| Normal lung tissue (lot. 0011-192-01283) | ND |
| Normal lung tissue (lot. 0011-192-01285) | ND |
| Normal lung tissue (lot. 0011-192-01297) | ND |
| The gene expression level was normalized by taking as 1 the median value of the expression levels of all genes that the expression was detected with the oligonucleotide microarray. | |
| ND: not detected | |

### EXAMPLE 2

### Apoptosis induction in human lung cancer cell line

The expression of SEMA4B gene and SEMA4B-M1, SEMA4B-M2 and SEMA4B-M3 genes described in EXAMPLE 4 was repressed to see if apoptosis was induced in human lung cancer cell line.

First, human non-small-cell lung cancer cell line NCI-H1703 purchased from American Type Culture Collection (ATCC) was suspended in RPMI-1640 medium (containing 25 mM HEPES) (Invitrogen Corp.) supplemented with 10% fetal calf serum (ATCC), and plated on a 96-well flat bottomed tissue culture plate (BD Falcon) at a cell density of 10,000 cells/well (0.1 ml of medium volume) and then incubated overnight at 37°C in a 5% carbon dioxide gas flow, followed by transfection of an antisense oligonucleotide.

Specifically, after the antisense oligonucleotide sequence (SEQ ID NO: 13) hybridizable to a sequence in the 3' untranslated region of the protein having the amino acid sequences represented by SEQ ID NO: 1, SEQ ID NO: 4, SEQ ID NO: 7 and SEQ ID NO: 10 was designed, phosphorothioated oligonucleotide was synthesized, purified on HPLC and provided for use in transfection experiment (hereinafter merely referred to as the antisense oligonucleotide). For control, the reverse sequence (SEQ ID NO: 14) of the base sequence shown by SEQ ID NO: 13 was similarly phosphorothioated, purified on HPLC and provided for use (hereinafter merely referred to as the control oligonucleotide).

The antisense oligonucleotide or the control oligonucleotide diluted in Opti-MEM (Invitrogen Corp.) was mixed with Oligofectamine (Invitrogen Corp.) diluted with Opti-MEM (Invitrogen Corp.) to 5-fold and settled at room temperature for 5 minutes, in a ratio of 8:3 (volume ratio). The resulting mixture was dispensed to the plate in 40 µL/well. The final concentration of the oligonucleotide was adjusted to become 250 nM. After incubation was continued for further 3 days under the conditions described above, the apoptosis induction activity of the two oligonucleotides above was assayed with Cell Death Detection ELISA^{PLUS} Kit (Roche Diagnostics) in accordance with the protocol attached thereto.

As a result, the antisense oligonucleotide (SEQ ID NO: 13) showed the apoptosis induction activity of approximately 1.6 times higher than the control oligonucleotide (SEQ ID NO: 14), indicating that there was a statistically significant difference (P≦0.01) (Table 3).

**[Table 3]**

| | Apoptosis Induction Activity (A₄₀₅-A₄₉₂) | |
|---|---|---|
| | Mean Value | Standard Deviation |
| Blank | 0.212 | 0.032 |
| Control oligonucleotide (SEQ ID NO: 14) | 0.410 | 0.017 |
| Antisense oligonucleotide (SEQ ID NO: 13) | 0.538 | 0.035 |

### EXAMPLE 3

### Reduction in gene expression level by SEMA4B antisense oligonucleotide

It was examined if the expression levels of SEMA4B gene and SEMA4B-M1, SEMA4B-M2 and SEMA4B-M3 genes described in EXAMPLE 4 were reduced by administration of the antisense oligonucleotide.

Human non-small-cell lung cancer cell line NCI-H1703 used in EXAMPLE 2 was suspended in the same medium as in EXAMPLE 2, and plated on a 24-well flat bottomed tissue culture plate (BD Falcon) at a cell density of 60,000 cells/well (0.6 ml of medium volume). The cells were incubated overnight at 37°C in a 5% carbon dioxide gas flow, followed by transfection of the antisense oligonucleotide. However, the oligonucleotide solution was added in a volume of 240 µL/well and two antisense oligonucleotides (SEQ ID NO: 13 and SEQ ID NO: 15) and two oligonucleotides (SEQ ID NO: 14 and SEQ ID NO: 16) for control were used.

Concerning the antisense oligonucleotide from SEQ ID NO: 15 and the control oligonucleotide from SEQ ID NO: 16, the antisense oligonucleotide sequence (SEQ ID NO: 15) hybridizable to a sequence in the 3' untranslated region of the protein having the amino acid sequences represented by SEQ ID NO: 1, SEQ ID NO: 4, SEQ ID NO: 7 and SEQ ID NO: 10 was designed. Then, the phosphorothioated oligonucleotide was synthesized, purified on HPLC and used for transfection experiment. The reverse sequence (SEQ ID NO: 16) of the base sequence represented by SEQ ID NO: 15 was similarly phosphorothioated, purified on HPLC and provided for use.

Following the transfection, incubation was continued at 37°C for further 24 hours in a 5% carbon dioxide gas flow and the total RNA was then extracted by RNeasy (registered trademark) Mini Total RNA Kit (QIAGEN). Using as a template about 300 ng of the total RNA, reverse transcription was carried out on TaqMan Reverse Transcription Reagents (Applied Biosystems) in accordance with the protocol attached thereto. Using as a template cDNA in an amount corresponding to 7 to 9 ng when converted into the total RNA, the number of expressed copies of SEMA4B, SEMA4B-M1, SEMA4B-M2 and SEMA4B-M3 genes was determined using two primers (SEQ ID NO: 17) and SEQ ID NO: 18) and SYBR Green PCR Master Mix (Applied Biosystems). The expression level of a gene for β-actin contained in the same amount of template cDNA was assayed on TaqMan β-actin Control Reagents (Applied Biosystems), which was used as internal standard.

When distilled water was used in place of the oligonucleotide solution (hereinafter briefly referred to as the non-transfection group), the total expression level of SEMA4B, SEMA4B-M1, SEMA4B-M2 and SEMA4B-M3 genes was 6.6% of the expression level of β-actin gene, whereas in the groups given with the antisense oligonucleotides (SEQ ID NO: 13 and SEQ ID NO: 15), the expression levels were 0.98% and 1.1%, indicating that a statistically significant (P ≦ 0.05) reduction in the expression level was observed.

On the other hand, the expression levels were 4.1% and 3.4% in the groups given with the control oligonucleotides (SEQ ID NO: 14 and SEQ ID NO: 16), indicating that any statistically significant reduction in the expression level was not observed when compared to the non-transfection group.

These results revealed that the repressed expression of SEMA4B, SEMA4B-M1, SEMA4B-M2 and SEMA4B-M3 genes were correlated to the induction of apoptosis.

### EXAMPLE 4

### Cloning and base sequencing of cDNAs encoding SEMA4B, SEMA4B-M1, SEMA4B-M2 and SEMA4B-M3

Using human lung cancer cell line (A549)-derived Marathon-Ready cDNA (CLONTECH) as a template, PCR was carried out by using two primers (SEQ ID NO: 19 and SEQ ID NO: 20). The reaction solution (50 µl) was composed of 1 µl of the above cDNA, 2.5 U of PfuTurbo Hotstart DNA Polymerase (STRATAGENE), 1.0 µM each of the primers (SEQ ID NO: 19 and SEQ ID NO: 20), 200 µM of dNTPs and 25 µl of 2 x GC Buffer I (Takara Shuzo Co., Ltd.). PCR was carried out by reacting at 95°C for 1 minute and then repeating 30 times the cycle set to include 95°C for 1 minute, 60°C for 1 minute and 72°C for 4 minutes, and extension was performed at 72°C for 5 minutes. In order to add dATP to the PRC product at the 3' end, 5U of Ex Taq DNA Polymerase (Takara Shuzo Co., Ltd.) was added and the mixture was kept at 72°C for 7 minutes. The PCR product obtained was purified using PCR Purification Kit (QIAGEN). The purified product was subcloned to plasmid vector pCR4-TOPO (Invitrogen Corp.) according to the protocol of TOPO TA PCR Cloning Kit (Invitrogen Corp.). The clones were transfected to Escherichia coli TOP10 and the clones bearing cDNA were selected in ampicillin-containing LB agar medium. The base sequences of individual clones were analyzed to give the base sequences of cDNAs represented by SEQ ID NO: 2, SEQ ID NO: 5, SEQ ID NO: 8 and SEQ ID NO: 11, respectively.

The base sequences in which the 1-237 base sequence and the 2749-3766 base sequence in the base sequence for SEMA4B gene (GenBank Accession No. XM_044533 gene) are added to the base sequences represented by SEQ ID NO: 2, SEQ ID NO: 5, SEQ ID NO: 8 and SEQ ID NO: 11 at the 5' and 3' ends thereof are shown by SEQ ID NO: 3, SEQ ID NO: 6, SEQ ID NO: 9 and SEQ ID NO: 12, respectively.

The amino acid sequence (SEQ ID NO: 1) encoded by the base sequence represented by SEQ ID NO: 2 completely coincided with SEMA4B protein encoded by SEMA4B gene (GenBank Accession No. XM_044533 gene).

The protein containing the amino acid sequence (SEQ ID NO: 4) encoded by the base sequence represented by SEQ ID NO: 5, the protein containing the amino acid sequence (SEQ ID NO: 7) encoded by the base sequence represented by SEQ ID NO: 8 and the protein containing the amino acid sequence (SEQ ID NO: 10) encoded by the base sequence represented by SEQ ID NO: 11 were named SEMA4B-M1, SEMA4B-M2 and SEMA4B-M3, respectively.

In the amino acid sequence (SEQ ID NO: 4) of SEMA4B-M1, Ser at the 208 position is replaced by Ile in the amino acid sequence (SEQ ID NO: 1) of SEMA4B.

In the base sequence (SEQ ID NO: 5) of DNA encoding SEMA4B-M1, g at the 90 position, g at the 111 position and g at the 623 position in the base sequence (SEQ ID NO: 2) of DNA encoding SEMA4B are replaced by a, a and t, respectively and the substitution at the 623 position is accompanied by amino acid substitution.

In the amino acid sequence (SEQ ID NO: 7) of SEMA4B-M2, Met at the 163 position is replaced by Ile in the amino acid sequence (SEQ ID NO: 1) of SEMA4B.

In the base sequence (SEQ ID NO: 8) of DNA encoding SEMA4B-M2, g at the 150 position, g at the 489 position, c at the 528 position, t at the 1266 position, c at the 1588 position and a at the 2343 position in the base sequence (SEQ ID NO: 2) of DNA encoding SEMA4B are replaced by a, a, t, c, a and g, respectively and the substitution at the 489 position is accompanied by amino acid substitution.

In the amino acid sequence (SEQ ID NO: 10) of SEMA4B-M3, Lys at the 364 position is replaced by Asn in the amino acid sequence (SEQ ID NO: 1) of SEMA4B.

In the base sequence (SEQ ID NO: 11) of DNA encoding SEMA4B-M3, g at the 1092 position in the base sequence (SEQ ID NO: 2) of DNA encoding SEMA4B is replaced by t, accompanied by amino acid substitution.

The plasmid bearing DNA having the base sequence represented by SEQ ID NO: 2, the plasmid bearing DNA having the base sequence represented by SEQ ID NO: 5, the plasmid bearing DNA having the base sequence represented by SEQ ID NO: 8 and the plasmid bearing DNA having the base sequence represented by SEQ ID NO: 11 were named SEMA4B/pCR4-TOPO, SEMA4B-M1/pCR4-TOPO, SEMA4B-M2/pCR4-TOPO and SEMA4B-M3/pCR4-TOPO, respectively.

Furthermore, the plasmid SEMA4B/pCR4-TOPO-transfected transformant, the plasmid SEMA4B-M1/pCR4-TOPO-transfected transformant, the plasmid SEMA4B-M2/pCR4-TOPO-transfected transformant and the plasmid SEMA4B-M3/pCR4-TOPO-transfected transformant were named Escherichia coli TOP10/SEMA4B/pCR4-TOPO, Escherichia coli TOP10/SEMA4B-M1/pCR4-TOPO, Escherichia coli TOP10/SEMA4B-M2/pCR4-TOPO and Escherichia coli TOP 10/SEMA4B-M3/pCR4-TOPO, respectively.

### EXAMPLE 5

### Study of gene expression level in human cell line

The following 86 strains of brain tumor cell lines SK-N-MC, SK-N-AS, SK-N-BE, SK-N-DZ, SK-N-FI, SK-N-SH, D341Med, Daoy, DBTRG-05MG, U-118 MG, U-87 MG, CCF-STTG1 and SW 1088; human breast cancer cell lines HCC1937, ZR-75-1, AU565, MCF-7 and MDA-MB-231; human colon cancer cell lines Caco-2, COLO201, COLO 205, COLO 320DM, HCT-8, HT-29, LoVo, LS123, SNU-C1, SK-CO-1, SW 403, SW 48, SW480, SW 620, SW 837 and SW 948; human embryonic kidney cell line HEK293; human small cell cancer cell lines NCI-H187, NCI-H378, NCI-H526, NCI-H889, NCI-H1672, NCI-H1836, NCI-H2227, NCI-N417 and SHP-77; human non-small cell lung cancer cell lines A549, NCI-H23, NCI-H226, NCI-H358, NCI-H460, NCI-H522, NCI-H661, NCI-H810, NCI-H1155, NCI-H1299, NCI-H1395, NCI-H1417, NCI-H1435, NCI-H1581, NCI-H1651, NCI-H1703, NCI-H1793, NCI-H1963, NCI-H2073, NCI-H2085, NCI-H2106, NCI-H2228, NCI-H2342 and NCI-H2347; human ovary cancer cell lines ES-2, Caov-3, MDAH2774, NIH:OVCAR3, OV-90, SK-OV-3, TOV-112D and TOV-21 G; human pancreatic cancer cell lines PANC-1, MIA-PaCa-2, AsPC-1, BxPC-3, Capan-1 and Capan-2; human prostate cancer cell lines DU145; human retinoblastoma cell line WERI-Rb-1 and Y79; and human testicular cancer cell line Cates-1B used below were purchased from ATCC. Human normal small airway epithelial cells SAEC and human normal prostate epithelial cells HPrEC were purchased from Clonetics Corp. Human colon cancer cell line COCM1, human non-small lung cancer cell line VMRC-LCD and human prostate cancer cell line PC3 were purchased from JCRB. These cell lines are sometimes used in EXAMPLE 9 and the following EXAMPLES.

Total RNA was prepared from the 91 cell lines described above using RNeasy Mini Total RNA Kit (QIAGEN). Reverse transcription was performed on the total RNA as a template using a random primer to prepare cDNA. Using this cDNA as a template, quantitative PCR was carried out to examine the expression levels of SEMA4B gene (SEQ ID NO: 2), SEMA4B-M1 gene (SEQ ID NO: 5), SEMA4B-M2 gene (SEQ ID NO: 8) and SEMA4B-M3 gene (SEQ ID NO: 11).

In the PCR above, the reaction was carried out under the same conditions as in EXAMPLE 3, using cDNA obtained from 3 to 4 ng of the total RNA described above as the template, and the copies of SEMA4B, SEMA4B-M1, SEMA4B-M2 and SEMA4B-M3 genes expressed were calculated. In parallel, the copy number of the gene for β-actin contained in 1 ng of the total RNA above was calculated using TaqMan™ Human β-actin Control Reagents (Applied Biosystems) and used as an internal standard.

A relative expression rate obtained by normalizing the total gene expression level described above with the gene expression level of β-actin is shown in Table 4.

The cancer cell lines in which the total gene expression level described above exceeds 1% of the gene expression level of β-actin gene were found to be 17 strains, indicating that enhanced expression of the genes above was noted in the cancer cell lines.

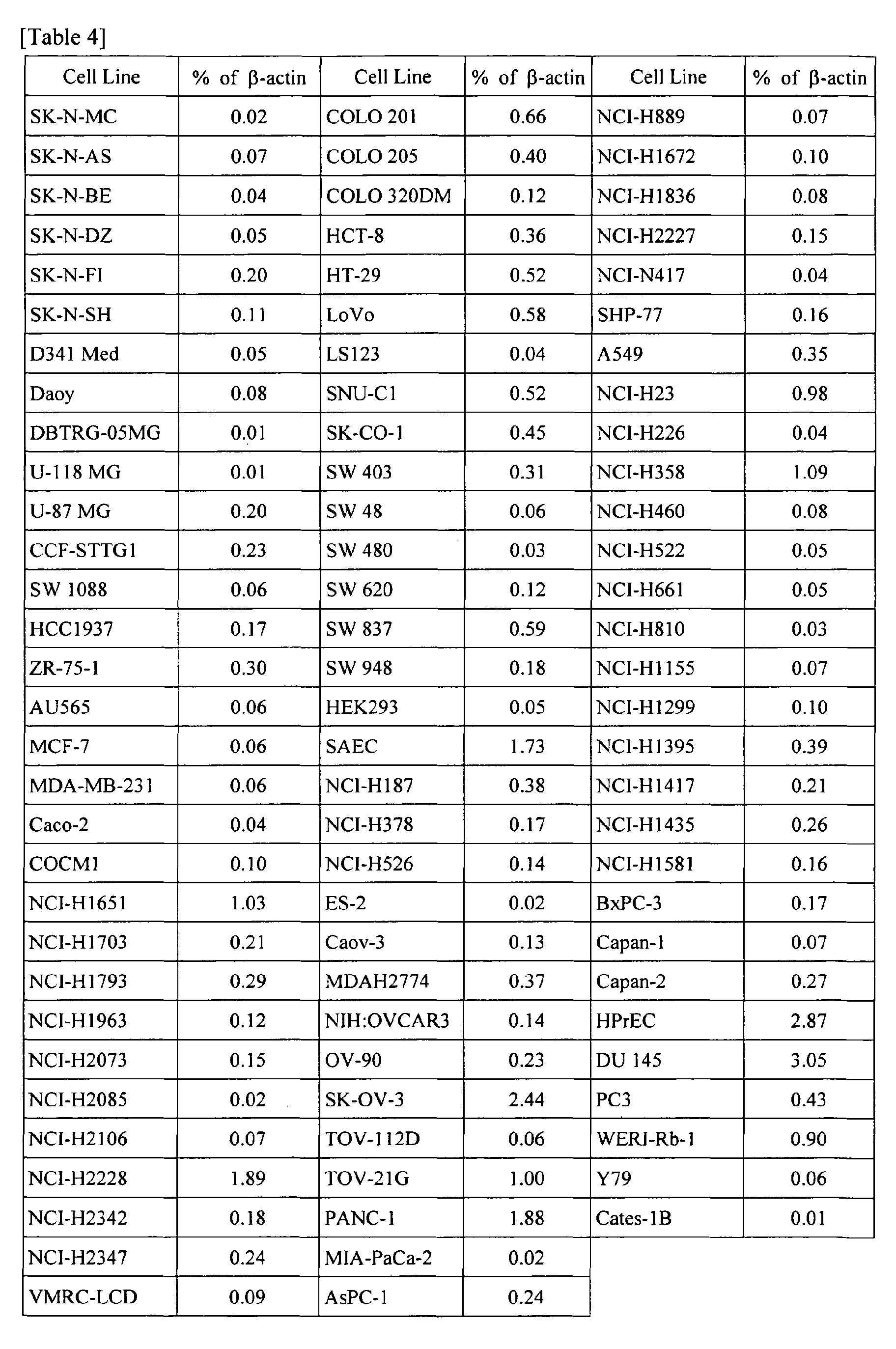

### EXAMPLE 6

### Construction of animal cell expression vectors for recombinant full-length protein

SEMA4B gene was amplified by PCR using the plasmid SEMA4B/pCR4-TOPO obtained in EXAMPLE 4 as a template. In the reaction solution for the reaction, 2 ng of SEMA4B/pCR4-TOPO was used as a template and 2.5 U of Pfu Turbo Hotstart DNA Polymerase (STRATAGENE), 1 µM each of 2 primers (SEQ ID NO: 19 and SEQ ID NO: 21), 200 µM of dNTPs and 5 µl of 10 x Pfu Buffer were added to make the solution volume 50 µl. PCR was carried out by reacting at 95°C for 1 minute and then repeating 25 times the cycle set to include 95°C for 1 minute, 60°C for 1 minute and 72°C for 4 minutes. Next, the PCR product was purified using PCR Purification Kit (QIAGEN) and then treated with restriction enzymes XbaI and Eco RI. The plasmid p3xFLAG-CMV-14 (Sigma) was also treated with XbaI and Eco RI. Each DNA fragment was purified on PCR Purification Kit, followed by ligation using DNA Ligation Kit ver.2 (Takara Bio, Inc.). After the ligation solution was transfected to Escherichia coli TOP 10, the transformed Escherichia coli was selected in ampicillin-containing LB agar medium. As a result of the analysis of individual clones, the plasmid pCMV-14- SEMA4B bearing the cDNA fragment corresponding to SEMA4B gene (SEQ ID NO: 2) was obtained.

### EXAMPLE 7

### Construction of animal cell expression vector for recombinant full-length protein with recombinant tag

Animal cell expression vector capable of expressing SEMA4B protein fused with 3xFLAG tag at the C terminus of the protein was constructed. A pair of primers used to amplify SEMA4B gene by PCR were changed to another pair of primers (SEQ ID NO: 19 and SEQ ID NO: 20) and otherwise under the same conditions as in the method described in EXAMPLE 6, the transformed Escherichia coli was selected. As a result, the plasmid pCMV-14-SEMA4B-3xFLAG bearing the cDNA fragment encoding the SEMA4B protein (SEQ ID NO: 1) fused with 3xFLAG tag at the C terminus of the protein to SEMA4B gene (SEQ ID NO: 2) was obtained.

### EXAMPLE 8

### Production and purification of peptide antibodies

Based on the amino acid sequences of SEMA4B protein (SEQ ID NO: 1), SEMA4B-M1 protein (SEQ ID NO: 4), SEMA4B-M2 protein (SEQ ID NO: 7) and SEMA4B-M3 protein (SEQ ID NO: 10), the following 4 peptides (Peptides 1 to 4) composed of 12 to 15 amino acids were synthesized by the Fmoc solid phase synthesis.

The amino acid sequence of Peptide 1 [Asn-Ser-Ala-Arg-Glu-Arg-Lys-Ile-Asn-Ser-Ser-Cys (SEQ ID NO: 22)] is a sequence of the 402-412 amino acid sequence in SEMA4B protein (SEQ ID NO: 1), in which Cys is added to the amino acid sequence at the C terminus.

The amino acid sequence of Peptide 2 [Ser-Val-Val-Ser-Pro-Ser-Phe-Val-Pro-Thr-Gly-Glu-Lys-Pro-Cys (SEQ ID NO: 23)] is a sequence of the 582-596 amino acid sequence in SEMA4B protein (SEQ ID NO: 1).

The amino acid sequence of Peptide 3 [Pro-Leu-Asp-His-Arg-Gly-Tyr-Gln-Ser-Leu-Ser-Asp-Ser-Pro-Cys(SEQ ID NO: 24)] is a sequence of the 781-794 amino acid sequence in SEMA4B protein (SEQ ID NO: 1), in which Cys is added to the amino acid sequence at the C terminus.

The amino acid sequence of Peptide 4 [Ser-Arg-Val-Phe-Thr-Glu-Ser-Glu-Lys-Arg-Pro-Leu-Ser-Cys (SEQ ID NO: 25)] is a sequence of the 797-809 amino acid sequence in SEMA4B protein (SEQ ID NO: 1), in which Cys is added to the amino acid sequence at the C terminus.

Keyhole limpet hemocyanin (KLH) as a carrier protein was coupled to the respective peptides of Peptides 1, 2, 3 and 4, which were used as antigens to produce rabbit polyclonal antibodies, as described below.

One male rabbit KBL: JW (11weeks old, Oriental Yeast Co., Ltd.) was used as an immunized animal. A suspension of complete Freund's adjuvant (Difco Laboratories) was used for primary sensitization and a suspension of incomplete adjuvant (Difco Laboratories) for the second sensitization and thereafter. The sensitization was performed by subcutaneous injection at the back and 0.5 mg of each antigen was used per sensitization. After the primary sensitization, it was repeated 3 times every 14 days. On day 52 after the primary sensitization, blood was collected through the carotid artery under anesthesia to give about 50 ml of serum. The serum thus obtained was concentrated by means of ammonium sulfate salting out. The total amount of the crude IgG fractions obtained were purified on protein A-affinity column (Amersham-Bioscience Corp.) to give about 103 mg, about 76 mg, about 112 mg and about 122 mg of purified IgGs from Peptides 1, 2, 3 and 4, respectively. Furthermore, the IgG fractions bound to a column immobilized with the respective immunogen peptides were acquired. For the immobilization, the C-terminal Cys of each peptide was utilized and the peptide was coupled to Sepharose column (Amersham-Bioscience Corp.) using borate buffer. For elution from the column, 8M urea/phosphate buffered saline (PBS) was used. The eluate was dialyzed to PBS to remove urea, which was followed by ultraconcentration and sterilization by filtering. Thus, affinity-purified antibodies AS-2531, AS-2532, AS-2591 and AS-2592 to Peptides 1, 2, 3 and 4 were acquired in about 15 mg, about 126 mg, about 17 mg and about 35 mg, respectively.

### EXAMPLE 9

### Western blotting using rabbit peptide antibodies

SEMA4B protein (SEQ ID NO: 1) was detected using the purified peptide antibodies prepared in EXAMPLE 8. Human non-small lung cancer-derived NCI-H358 cells were suspended in 10 ml ofRPMI-1640 medium (Invitrogen Corp.) containing 10% fetal calf serum (JRH) at a concentration of 1.5 x 10⁶ and plated on a Petri dish of 10 cm in diameter. After incubation at 37°C overnight in a 5% carbon dioxide flow, 6 µg of the plasmid pCMV-14- SEMA4B prepared in EXAMPLE 6 was mixed with Plus reagent (Invitrogen Corp.) and OPTI-MEM I (Invitrogen Corp.). After the mixture was allowed to stand at room temperature for 15 minutes, LipofectAMINE Transfection Reagent (Invitrogen Corp.) and OPTI-MEM I were added to the mixture, which was allowed to stand at room temperature for further 15 minutes. The resulting mixture was dropwise added to the medium and incubation was continued. Two days after the transfection of expression plasmid, the cells were washed with ice-cooled PBS and 1 ml of ice-cooled RIPA buffer [50 mM Tris-hydrochloride buffer, pH 7.5, 150 mM sodium chloride, 1% Triton X-100, 0.1% SDS, 1% deoxycholic acid, Complete™ tablet (Roche Diagnostics), Phosphatase Inhibitor Cocktail-2 (Sigma)] was added to the cells. The mixture was allowed to stand at 4°C for 30 minutes. This RIPA buffer was recovered and centrifuged at 15,000 rpm for 20 minutes. The supernatant obtained was used as the cell-free extract. This cell-free extract was mixed with a 2-fold concentration of SDS-PAGE sample buffer [125 mM Tris-hydrochloride buffer, pH 6.8, 40% glycerol, 4% SDS, 0.04% Bromophenol Blue and 5% 2-mercaptoethanol] in equal volumes. After heating at 95°C for 5 minutes, 10 µl of the mixture was provided for SDS-PAGE on 10% acrylamide gel. The protein separated by electrophoresis was transferred onto Clear Blotting P Membrane (ATTO) in a conventional manner, which was then allowed to stand in a blocking buffer [50 mM Tris-hydrochloride buffer, pH 7.5, 500 mM sodium chloride, 0.1% Tween 20, 5% skimmed milk] at room temperature for an hour. Next, the peptide antibody AS-2531, AS-2532, AS-2591 or AS-2592 produced in EXAMPLE 8 were diluted with the blocking buffer in a concentration of 3 µg/ml, followed by reacting at 4°C overnight. Subsequently, the reaction mixture was allowed to stand for an hour in a dilution of HRP-labeled anti-rabbit IgG antibody (Amersham-Bioscience Corp.) diluted in the blocking buffer to 50,000-fold or 100,000-fold. Detection was performed according to the protocol attached to ECL plus (Amersham-Bioscience Corp.). Thus, the SEMA4B protein was detected.

Even when any of AS-2532, AS-2591 and AS-2592 except AS-2531 was used, a specific band attributed to the SEMA4B protein was noted at the position near 100 kD molecular weight.

### EXAMPLE 10

### Immunoprecipitation using the rabbit peptide antibodies

Using the purified peptide antibodies produced in EXAMPLE 8, immunoprecipitation was performed on the SEMA4B protein under non-denaturing conditions.

Using the plasmid pCMV-14-SEMA4B-3xFLAG acquired in EXAMPLE 7, the cell-free extract was prepared by the same procedures as in EXAMPLE 9. The cell-free extract, 400 µl, was added to 50 µl of a suspension of Protein G-Sepharose 4FF (Amersham-Bioscience Corp.) prepared by suspending in an equal volume of RIPA buffer) and 5 µg of any one of the peptide antibodies AS-2531, AS-2532, AS-2591 and AS-2592 described in EXAMPLE 8 was further added thereto. The resulting mixture was agitated at 4°C overnight. After the Protein G-Sepharose 4FF co-precipitated fraction was washed with RIPA buffer, the fraction was suspended in 50 µl of SDS-PAGE sample buffer [62.5 mM Tris-hydrochloride buffer, pH 6.8, 20% glycerol, 2% SDS, 0.02% Bromophenol Blue and 2.5% 2-mercaptoethanol]. After heating at 95°C for 5 minutes, 5 µl or 10 µl of the suspension was provided for SDS-PAGE on 10% acrylamide gel. Detection was performed by the same procedures as in EXAMPLE 9, except that mouse anti- FLAG M2 antibody (Sigma) diluted with the blocking buffer to 0.2 µg/ml or 0.1 µg/ml was used as a primary antibody and HRP-labeled anti-mouse IgG antibody (Amersham-Bioscience Corp.) diluted with the blocking buffer to 25,000-fold or 50,000-fold was used as a secondary antibody.

Even when immunoprecipitation was performed using any of the peptide antibodies AS-2531, AS-2532, AS-2591 and AS-2592, a specific band attributed to the SEMA4B protein was noted at the position near 100 kD molecular weight.

The results reveal that the peptide antibodies AS-2531, AS-2532, AS-2591 and AS-2592 bind to the non-denaturing SEMA4B protein.

### EXAMPLE 11

### Study of expression of SEMA4B protein in cancer cell lines

Lung cancer cell lines NCI-H2228, NCI-H1651, NCI-H358, NCI-H23 and NCI-H1703; ovary cancer cell lines SKOV-3 and TOV-21G; prostate cancer cell line DU145; and pancreatic cancer cell line PANC-1 were plated, respectively, on two Petri dishes of 10 cm in diameter. For each of the cells, the cells for one Petri dish were dispersed in Trypsin-EDTA and the number of cells was counted. Based on the cells counted, ice-cooled RIPA buffer (described in EXAMPLE 9) was added to the remaining one Petri dish in 1 ml/5 x 10⁶ cells, followed by allowing to stand at 4°C for 30 minutes. This RIPA buffer was recovered and centrifuged at 15,000 rpm for 20 minutes. The supernatant obtained was used as the cell-free extract. Meanwhile, a resin obtained by crosslinking the peptide antibody AS-2531 described in EXAMPLE 8 with Protein G-Sepharose 4FF (Amersham-Bioscience Corp.) according to the protocol attached to Size™ X Protein G Immunoprecipitation Kit (Pierce Chemical) was prepared and suspended in an equal volume of RIPA buffer. The aforesaid cell-free extract, 400 µl, was added to 30 µl of this suspension, followed by agitation overnight at 4°C. After washing the Protein G-Sepharose 4FF co-precipitated fraction with RIPA buffer, the fraction was suspended in 30 µl of SDS-PAGE sample buffer described in EXAMPLE 10 and the suspension was heated at 95°C for 5 minutes. Then, 20 µl of the suspension was provided for SDS-PAGE on 10% acrylamide gel. Using the peptide antibody AS-2532, detection was performed in a manner similar to EXAMPLE 9.

In the 9 cell lines described above, a specific band attributed to the SEMA4B protein was observed at the position near 100 kD molecular weight in each cell line of NCI-H2228, NCI-H358, NCI-H23, SKOV-3, DU145 and PANC-1. The results reveal that the SEMA4B protein is overexpressed in the 6 cancer cell lines described above.

### EXAMPLE 12

### Establishment of the cell line stably expressing the full-length recombinant protein

Human non-small cell lung cancer-derived NCI-H358 was suspended in 2 ml of RPMI-1640 medium (Invitrogen Corp.) containing 10% fetal calf serum (JRH), 1 mM sodium pyruvate and 25 mM HEPES. The suspension was plated on a 6-well plate, followed by incubation overnight at 37°C in a 5% carbon dioxide gas. On the other hand, 1 µg of plasmid pCMV-14-SEMA4B described in EXAMPLE 6, which was diluted with the OPTI-MEM I (Invitrogen Corp.), was mixed with 6 µl of Plus reagent (Invitrogen Corp.) and the mixture was allowed to stand at room temperature for 15 minutes. Then, 4 µl of LipofectAMINE reagent (Invitrogen Corp.) diluted in OPTI-MEM I was added to the mixture, which was allowed to stand at room temperature for further 15 minutes. The mixture was dropwise added to the medium and incubation was further continued for a day. The cells were then dispersed in trypsin-EDTA (Invitrogen Corp.) and diluted to 10-fold in the above medium added with G418 (Promega Corp.) in 400 µg/ml, followed by plating which was plated on a 24-well plate. While the medium was exchanged with the G418-containing medium (G418 selection medium) every 3 or 4 other days, incubation was continued at 37°C in a 5% carbon dioxide gas flow. From colonies formed when one to three cells proliferated, the cells were recovered and plated equally on two wells of a 48-well plate. After incubation was continued until the cell density reached 50% or more, 50 µl of the SDS-PAGE sample buffer described in EXAMPLE 10 was added to the cells for one well to prepare the cell lysate. After heat treatment at 95°C for 5 minutes, 5 µl was provided on for SDS-PAGE on 10% acrylamide gel. Using the peptide antibody AS-2532, western blotting was performed by a modification of the procedures described in EXAMPLE 9 to explore a stable cell line constitutively expressing the SEMA4B-A protein (SEQ ID NO: 1). The cells recovered from the other well were diluted in 0.7 cell/well and then plated on a 96-well plate. While exchanging the G418 selection medium every 3 or 4 other days, incubation was continued at 37°C in a 5% carbon dioxide gas flow until the cell density reached about 50%. Again, the cells were plated equally on 2 wells of a 48-well plate, and incubation was continued until the cell density reached 50% or more. Using the cell lysate prepared from the cells for one well, western blotting was performed as described above. A clone with the highest expression of SEMA4B protein (SEQ ID NO: 1) was selected to acquire SEMA4B/H358 as the cell line stably expressing SEMA4B.

### EXAMPLE 13

### Study of localization of SEMA4B protein (biotin labeling)

Using non-small cell lung cancer cell lines NCI-H2228 and NCI-H358 and the cell line (SEMA4B/H358) stably expressing the full-length recombinant protein prepared in EXAMPLE 12, the proteins exposed on the cell surfaces were biotinylated with Cellular Labeling and Immunoprecipitation Kit (Roche Diagnostics). Subsequently, the cell-free extract was prepared by the procedures of EXAMPLE 9. Using 1 ml of the cell-free extract thus prepared and 5 µg of the peptide antibody AS-2591 prepared in EXAMPLE 8, immunoprecipitation was performed in accordance with the process of EXAMPLE 10, followed by SDS-PAGE. By detection with HRP-labeled streptoavidin (Amersham-Bioscience Corp.), bands attributed to the SEMA4B protein were noted near 100 kD molecular weight. This reveals that the SEMA4B protein, SEMA4B-M1 protein, SEMA4B-M2 protein and SEMA4B-M3 protein are localized on the cell surface.

### EXAMPLE 14

### Study of localization of SEMA4B protein (FACS analysis)

Human non-small cell lung cancer cell lines NCI-H2228 and NCI-H358 and SEMA4B/H358 described in EXAMPLE 12 were plated, respectively, on a Petri dish of 10 cm in diameter and incubated to become subconfluent. After the respective cells were washed with PBS, PBS containing 0.5% BSA and 5 mM EDTA were added thereto. The mixture was allowed to stand at room temperature for 15 minutes to disperse the cells. Next, the cells were suspended in Buffer A [HBSS (Hanks' Balanced Salt Solutions, Invitrogen Corp.) containing 2% fetal calf serum (JRH) and 0.1% sodium azide] in a concentration of 4 x 10⁶/ml, and AS-2532 or non-immunized rabbit IgG (Jackson) was added to the suspension in a final concentration of 10 µg/ml. The mixture was allowed to stand in ice for 3 hours. The cells were then washed with Buffer A and suspended in Buffer A containing 10 µg/ml of Alexa488-labeled anti-rabbit IgG antibody (Molecular Probes), followed by allowing to stand on ice for 2 hours. After washing again with Buffer A, the cells were analyzed by FACScan (BD Biosciences). The results reveal that all cells were stained specifically to rabbit peptide antibody AS-2532, indicating that the SEMA4B protein, SEMA4B-M1 protein, SEMA4B-M2 protein and SEMA4B-M3 protein are localized on the cell surface.

### EXAMPLE 15

### Apoptosis induction of human non-small cell lung cancer cell line NCI-H358 by transfection of the antisense oligonucleotide

It was examined if apoptosis could be induced also in human non-small cell lung cancer cell line other than NCI-H1703 described in EXAMPLE 2 by transfection of the antisense oligonucleotide.

NCI-H358 was suspended in RPMI-1640 medium (Invitrogen Corp.) containing 10% fetal calf serum (JRH), 1 mM sodium pyruvate and 25 mM HEPES. NCI-H358 was plated on a 96-well flat bottomed tissue culture plate (BD Falcon) at a cell density of 8 x 10³/well (80 µl of medium volume), followed by incubation at 37°C overnight in a 5% carbon dioxide gas flow. On the other hand, 0.06 µg each of the oligonucleotide (SEQ ID NO: 13) described in EXAMPLE 2 and control oligonucleotide (SEQ ID NO: 14) were diluted in OPTI-MEM I (Invitrogen Corp.). The dilution was mixed with 0.5 µl of Plus reagent (Invitrogen Corp.) and the mixture was allowed to stand at room temperature for 15 minutes. To the mixture, 0.4 µl of LipofectAMINE transfection reagent (Invitrogen Corp.) diluted in OPTI-MEM I was added. The mixture was allowed to stand at room temperature for further 15 minutes. The whole volume of the mixture was added to the medium for NCI-H358, and incubation was continued for further 3 hours. Following the protocols attached to Cell Death Detection ELISA^{PLUS} (Roche Diagnostics) and Caspase-Glo 3/7 assay (Promega Corp.), the oligonucleotide described above was assayed for its apoptosis induction activity.

As a result, the oligonucleotide showed the apoptosis induction activity in NCI-H358 as higher by 1.42 times and 1.77 times, respectively, than the control antisense oligonucleotide used as a negative control by both Cell Death Detection ELISA^{PLUS} and Caspase-Glo 3/7 assay, indicating that there was a statistically significant difference (P≦0.01) (Tables 5 and 6).

**[Table 5]**

| | Apoptosis Induction Activity (A₄₀₅-A₄₉₂) | |
|---|---|---|
| | Mean Value | Standard Deviation |
| Blank | 0.217 | 0.007 |
| Control oligonucleotide (SEQ ID NO: 14) | 0.330 | 0.041 |
| Antisense oligonucleotide (SEQ ID NO: 13) | 0.467 | 0.029 |

**[Table 6]**

| | Apoptosis Induction Activity (CPS) | |
|---|---|---|
| | Mean Value | Standard Deviation |
| Blank | 7625 | 235 |
| Control oligonucleotide (SEQ ID NO: 14) | 8727 | 188 |
| Antisense oligonucleotide (SEQ ID NO: 13) | 15452 | 570 |

### EXAMPLE 16

### Apoptosis induction of human non-small cell cancer cell lines NCI-H2228, NCI-H1651 and NCI-H23 by transfection of antisense oligonucleotide

It was examined if apoptosis could also be induced in human non-small cell lung cancer cell lines other than NCI-H1703 (EXAMPLE 2) and NCI-H358 (EXAMPLE 15) by transfection of the antisense oligonucleotide.

For NCI-H2228, RPMI-1640 medium (Invitrogen Corp.) containing 10% fetal calf serum (JRH), 1 mM sodium pyruvate and 25 mM HEPES was used. For NCI-H1651, ACL-4 medium (ATCC) containing 10%FBS was used. For NCI-H23, RPMI-1640 medium (Invitrogen Corp.) containing 10% fetal calf serum (JRH) and 25 mM HEPES was used. The respective cells were suspended in the corresponding media and plated on a 96-well flat bottomed tissue culture plate (BD Falcon) at cell densities of 7.5 x 10³/well (NCI-H2228), 7.5 x 10³/well (NCI-H1651) and 5 x 10³/well (NCI-H23), respectively (125 µl of medium volume), followed by incubation overnight at 37°C in a 5% carbon dioxide gas flow. On the other hand, 0.135 µg each of the antisense oligonucleotide (SEQ ID NO: 13) described in EXAMPLE 2 and the control oligonucleotide (SEQ ID NO: 14) were diluted in OPTI-MEM I (Invitrogen Corp.), respectively. After each dilution was mixed with 0.75 µl of Plus reagent (Invitrogen Corp.), the mixture was allowed to stand at room temperature for 15 minutes. Then, 0.4 µl of LipofectAMINE reagent (Invitrogen Corp.) diluted in OPTI-MEM I was added to the mixture, which was allowed to stand at room temperature for further 15 minutes. The whole volume of the mixture was added to the medium and incubation was further continued for 3 days. Following the protocol attached to Cell Death Detection ELISA^{PLUS} (Roche Diagnostics), the oligonucleotide described above was assayed for its apoptosis induction activity.

As a result, the oligonucleotide showed the apoptosis induction activity in any cell line as higher by 1.58 times (NCI-H2228), 1.21 times (NCI-H1651) and 1.25 times (NCI-H23), respectively, than the control antisense oligonucleotide used as a negative control, wherein P-values were calculated to be P≦0.05 (NCI-H2228), P≦ 0.05 (NCI-H1651) and P≦ 0.01 (NCI-H23), showing a statistically significant difference (Tables 7, 8 and 9).

**[Table 7]**

| | Apoptosis Induction Activity (A₄₀₅-A₄₉₂) | |
|---|---|---|
| | Mean Value | Standard Deviation |
| Blank | 0.312 | 0.009 |
| Control oligonucleotide (SEQ ID NO: 14) | 0.526 | 0.043 |
| Antisense oligonucleotide (SEQ ID NO: 13) | 0.829 | 0.123 |

**[Table 8]**

| | Apoptosis Induction Activity (A₄₀₅-A₄₉₂) | |
|---|---|---|
| | Mean Value | Standard Deviation |
| Blank | 0.523 | 0.091 |
| Control oligonucleotide (SEQ ID NO: 14) | 1.152 | 0.101 |
| Antisense oligonucleotide (SEQ ID NO: 13) | 1.390 | 0.104 |

**[Table 9]**

| | Apoptosis Induction Activity (A₄₀₅-A₄₉₂) | |
|---|---|---|
| | Mean Value | Standard Deviation |
| Blank | 0.678 | 0.028 |
| Control oligonucleotide (SEQ ID NO: 14) | 1.081 | 0.050 |
| Antisense oligonucleotide (SEQ ID NO: 13) | 1.351 | 0.058 |

### EXAMPLE 17

### Apoptosis induction using rabbit peptide antibodies

Human non-small lung cancer cell line NCI-H2228 was treated with rabbit peptide antibodies AS-2531 and AS-2532 acquired in EXAMPLE 8 and the apoptosis induction activities of these rabbit peptide antibodies were assayed.

NCI-H2228 was suspended in RPMI-1640 medium (Invitrogen Corp.) containing 10% fetal calf serum (JRH), 1 mM sodium pyruvate and 25 mM HEPES. The suspension was plated on a 96-well flat-bottomed tissue culture plate (BD Falcon) coated with type I collagen to reach a cell density of 4 x10³/well, followed by incubation overnight at 37°C in a 5% carbon dioxide gas. The rabbit peptide antibodies AS-2531 and AS-2532 acquired in EXAMPLE 8 and non-immunized rabbit IgG (Jackson) were diluted in PBS. The suspensions were added to the media, whereby the final concentrations of the antibodies reached 15 µg/ml, 45 µg/ml and 150 µg/ml, respectively. After incubation was continued for further 5 days, the rabbit peptide antibodies described above were assayed for their apoptosis induction activities, following the protocol attached to Cell Death Detection ELISA^{PLUS} (Roche Diagnostics).

As a result, the peptide antibodies showed the apoptosis induction activity in the presence of 45 µg/ml and 15 µg/ml of AS-2531 as higher by 1.26 times and 1.31 times, respectively, than the non-immunized rabbit IgG of the same concentration (P ≦0.05 and P≦0.01). Also, in the presence of 150 µg/ml of AS-2532, the peptide antibodies showed the apoptosis induction activity as higher by 1.27 times than the non-immunized rabbit IgG of the same concentration (P≦0.01).

As such, it became clear that the SEMA4B protein, SEMA4B-M1 protein, SEMA4B-M2 protein and SEMA4B-M3 protein play an important role in sustaining the survival of human lung cancer cells.

### INDUSTRIAL APPLICABILITY

The protein used in the present invention is specifically expressed in cancer cells and is a diagnostic marker for cancer. Thus, the compound or its salt that inhibits the activity of said protein, the compound or its salt that inhibits the expression of a gene for the protein, the antisense polynucleotide of the present invention and the antibody of the present invention can be safely used as an agent for preventing/treating a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), as an apoptosis promoter (inducer), etc. In addition, the protein used in the present invention, the polynucleotide encoding the protein, the antibody of the present invention, etc. are useful for screening an agent for preventing/treating a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), an apoptosis promoter (inducer), etc.

## Claims

1. A protein comprising the same or substantially the same amino acid sequence as the amino acid sequence represented by SEQ ID NO: 4, SEQ ID NO: 7 or SEQ ID NO: 10, or a salt thereof.

2. A protein consisting of the amino acid sequence represented by SEQ ID NO: 4, SEQ ID NO: 7 or SEQ ID NO: 10, or a salt thereof.

3. A partial peptide of the protein according to claim 1, or a salt thereof.

4. A polynucleotide comprising a polynucleotide encoding the protein according to claim 1, or a partial peptide thereof.

5. The polynucleotide according to claim 4, which is a DNA.

6. The polynucleotide according to claim 5, which contains a base sequence represented by SEQ ID NO: 5, SEQ ID NO: 8 or SEQ ID NO: 11.

7. A polynucleotide consisting of a base sequence represented by SEQ ID NO: 5, SEQ ID NO: 8 or SEQ ID NO: 11.

8. A recombinant vector comprising the polynucleotide according to claim 4.

9. A transformant transformed by the recombinant vector according to claim 8.

10. A method of manufacturing the protein according to claim 1, its partial peptide, or a salt thereof, which comprises culturing the transformant according to claim 9, and producing/accumulating the protein according to claim 1 or its partial peptide.

11. A pharmaceutical comprising the protein according to claim 1, its partial peptide, or a salt thereof.

12. A pharmaceutical comprising the polynucleotide according to claim 4.

13. A diagnostic agent comprising the polynucleotide according to claim 4.

14. An antibody to the protein according to claim 1, its partial peptide, or a salt thereof.

15. A pharmaceutical comprising the antibody according to claim 14.

16. A diagnostic agent comprising the antibody according to claim 14.

17. A polynucleotide comprising the entire or part of a base sequence complementary or substantially complementary to the polynucleotide according to claim 4.

18. A pharmaceutical comprising the polynucleotide according to claim 17.

19. A method of quantifying the protein according to claim 1, which comprises using the antibody according to claim 14.

20. A method for diagnosis of a disease associated with the protein according to claim 1 or with its function, which comprises using the quantifying method according to claim 19.

21. A method of screening a compound or its salt that inhibits the expression of the protein according to claim 1, which comprises using the protein according to claim 1, the partial peptide, or a salt thereof.

22. A kit for screening a compound or its salt that inhibits the expression of the protein according to claim 1, comprising the protein according to claim 1, the partial peptide, or a salt thereof.

23. A method of screening a compound or its salt that inhibits the expression of a gene for the protein according to claim 1, which comprises using the polynucleotide according to claim 4.

24. A kit for screening a compound or its salt that inhibits the expression of a gene for the protein according to claim 1, comprising the polynucleotide according to claim 4.

25. The pharmaceutical according to claim 11, 12, 15 or 18, which is a prophylactic/therapeutic agent for a cancer.

26. The pharmaceutical according to claim 11, 12, 15 or 18, which is an apoptosis promoter.

27. The diagnostic agent according to claim 13 or 16, which is a diagnostic agent for a cancer.

28. An apoptosis promoter comprising a substance that inhibits the expression of the protein according to claim 1 or a partial peptide thereof, or the expression of a gene for said protein.

29. An apoptosis promoter comprising an antibody to a protein comprising the same or substantially the same amino acid sequence as the amino acid sequence represented by SEQ ID NO: 1, its partial peptide, or a salt thereof.

30. A prophylactic/therapeutic agent for a cancer, comprising an antibody to a protein comprising the same or substantially the same amino acid sequence as the amino acid sequence represented by SEQ ID NO: 1, its partial peptide, or a salt thereof.

31. A polynucleotide comprising the entire or part of a base sequence complementary or substantially complementary to a base sequence of a polynucleotide encoding a protein comprising the same or substantially the same amino acid sequence as the amino acid sequence represented by SEQ ID NO: 1, or a partial peptide thereof.

32. A pharmaceutical comprising the polynucleotide according to claim 31.

33. The pharmaceutical according to claim 32, which is an apoptosis promoter.

34. A method of screening an apoptosis promoter, which comprises using a polynucleotide encoding a protein comprising the same or substantially the same amino acid sequence as the amino acid sequence represented by SEQ ID NO: 1, or a partial peptide thereof.

35. A kit for screening an apoptosis promoter, comprising a polynucleotide encoding a protein comprising the same or substantially the same amino acid sequence as the amino acid sequence represented by SEQ ID NO: 1, or a partial peptide thereof.

36. An apoptosis promoter, comprising a substance that inhibits the expression of a protein comprising the same or substantially the same amino acid sequence as the amino acid sequence represented by SEQ ID NO: 1, or its partial peptide, or the expression of a gene for said protein.

37. A method of preventing/treating a cancer, which comprises administering to a mammal an effective dose of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof.

38. A method of promoting apoptosis of cancer cells, which comprises administering to a mammal an effective dose of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof.

39. A method of preventing/treating a cancer, which comprises inhibiting the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, or inhibiting the expression of a gene for said protein or its partial peptide.

40. A method of promoting apoptosis of cancer cells, which comprises inhibiting the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, or inhibiting the expression of a gene for said protein or its partial peptide.

41. Use of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof, to manufacture a prophylactic/therapeutic agent for a cancer.

42. Use of (i) a substance that inhibits the expression of a protein comprising the same or substantially the same amino acid sequence as the amino acid sequence represented by SEQ ID NO: 1, SEQ ID NO: 4, SEQ ID NO: 7 or SEQ ID NO: 10, or its partial peptide, or a salt thereof, (ii) a substance that inhibits the expression of a gene for said protein or its partial peptide, or (iii) an antibody to said protein, its partial peptide, or a salt thereof, to manufacture an apoptosis promoter for cancer cells.
